# EUROPEAN PATENT APPLICATION

(11) **EP 4 548 864 A1**
(43) Date of publication of application: **07.05.2025**
(21) Application number: 23830427.3
(22) Date of filing: 29.06.2023
(51) Int. Cl.: A61B 17/16, A61B 17/56, A61B 34/30, A61F 2/46

(54) **HIP REPLACEMENT SURGERY ACTUATOR AND SURGICAL SYSTEM**

(30) Priority: 01.07.2022 CN 202210768533; 01.07.2022 CN 202210768534; 01.07.2022 CN 202210770077; 01.07.2022 CN 202210770082; 30.12.2022 CN 202211732816; 01.03.2023 CN 202310185951
(71) Applicant: BEIJING HURWA ROBOT MEDICAL TECHNOLOGY CO., LTD., Beijing 100176 (CN)
(72) Inventor: ZHANG, Zhao, Beijing 100176 (CN); ZHANG, Xiaofeng, Beijing 100176 (CN); LI, Wei, Beijing 100176 (CN); WANG, Chao, Beijing 100176 (CN); GUO, Jinbang, Beijing 100176 (CN); ZHANG, Chun, Beijing 100176 (CN)
(74) Representative: Murgitroyd & Company
(86) International application number: PCT/CN2023/103989
(87) International publication number: WO 2024/002262

(57) **Abstract**

Disclosed is an actuator for hip replacement, which is configured to prepare a space for installing a prosthesis on a bone and implanting the prosthesis. The actuator includes a first actuator and a second actuator. The first actuator is configured to connect a cutting tool to process an acetabulum and/or a medullary cavity, and the first actuator has a first connector and a second connector. The second actuator is configured to connect to the second connector of the first actuator during an operation of implantation of the prosthesis is performed, and connect the prosthesis and receive impact produced during installing the prosthesis. The actuator for the hip replacement is configured to be installed to a robotic arm via the first connector. During the hip joint surgery, when the acetabulum and the medullary cavity are prepared, the robotic arm is connected to the first actuator. When it is necessary to install the prosthesis, the second actuator is connected to the first actuator. By the above configuration, the operation of actuator replacement is capable to be reduced, thereby facilitating completing the hip replacement surgery.

## Description

### TECHNICAL FIELD

The present disclosure relates to a field of medical equipment, and in particular, to an actuator for hip replacement and a surgical system.

### BACKGROUND

Joint replacement surgery mainly comprises knee replacement and hip replacement. In total knee replacement, a distal end of a femur and a tibia that make up a knee joint need to be processed to form a shape and a size suitable for implanting a prosthesis. The processing to the femur and the tibia is mainly made by cutting with a saw, to form a plurality of planes. The precision of the implantation of the prosthesis of the knee joint is substantially determined by the shape of the processed bone, so that processing precision of each plane determines the precision of the implantation of the prosthesis. In total hip replacement, an acetabulum and a proximal end of the femur that make up a hip joint need to be processed to form a shape and a size suitable for implantation of a prosthesis. The forming of the hip joint includes grinding forming of an acetabular fossa, and osteotomy and reaming at a proximal end of the femur. The precision of the hip replacement involves the precision of the implantation of the prosthesis on a side of the acetabulum and the precision of the implantation of the prosthesis on a side of the femur. The precision of the implantation of the prosthesis on the side of the acetabulum depends on the processing precision of the acetabular fossa and control precision of the implanting angle and depth of the acetabular prosthesis during implanting. The precision of the implantation of the prosthesis on the side of the femur depends on the reaming precision on the side of the femur.

When the acetabular fossa is processed, it is necessary to use a grinding tool to grind the acetabular fossa. The grinding tool generally includes a hemispherical file head, a connecting rod with a certain length, a holding sleeve sleeved outside the connecting rod and a pistol-shaped power tool. One end of the connecting rod is connected to the file head, and the other end of the connecting rod is connected to a power output end of the pistol-shaped tool. In use, a surgeon holds a handle of the pistol-shaped power tool in one hand and holds the holding sleeve in the other hand, and then inserts the file head into the acetabulum and applies a force in an axial direction of the connecting rod to grind bone tissue on a surface of the acetabulum. In the grinding process, the surgeon controls an angle between the connecting rod and a pelvis and the depth of the grinding according to experience, to control the processing precision.

After the acetabular fossa is processed, it is necessary to use a cup holding apparatus to implant a prosthesis cup into the acetabulum. The cup holding apparatus includes a straight connecting rod or a connecting rod with an elbow, and a hammer. One end of the connecting rod is connected to the prosthesis cup, and the other end of the connecting rod is used for receiving hitting by the hammer. A middle portion of the connecting rod is used for holding by a surgeon. In use, the surgeon holds the middle portion of the connecting rod to control the angle of the connecting rod with respect to the pelvis. The other end of the connecting rod is hammered by the hammer to press the prosthesis cup into the acetabular fossa. During implanting, with each hammering, the whole cup holding apparatus will move axially with the prosthesis cup entering the acetabular fossa.

In recent years, technologies of using a robotic system to assist surgery are becoming gradually mature, such as a knee surgical robot sold by MAKO SURGICAL CORP. Generally, a robot system includes a robotic arm, a navigation and positioning system and a control system. The robotic arm is equivalent to an arm of a surgeon, which may hold a surgical tool and position the surgical tool with a relatively high precision. The navigation and positioning system is equivalent to an eye of the surgeon, which may measure a position of the surgical tool and a tissue of a patient in real time. The control system is equivalent to a brain of the surgeon, which stores a surgical plan therein. The control system calculates a route of the robotic arm and/or a position that the robotic arm to be reached according to information obtained by the navigation and positioning system during surgery, and may actively control movement of the robotic arm, or sets a virtual boundary for the robotic arm through a force feedback mode, and then manually push the robotic arm to move along a route, face or body defined by the virtual boundary. In a robot system of MAKO SURGICAL CORP, an electric swing saw is suspended at an end of a robotic arm. During surgery, the robotic arm positions the swing saw near a knee, and a surgeon operates to start and push the electric swing saw to cut a bone, thereby an installation position for implanting a prosthesis is prepared. Compared with traditional knee replacement, robot-assisted knee replacement has many advantages. For example, dependence on experience of a surgeon is reduced, and iatrogenic injuries caused by use of traditional mechanical positioning structures is reduced.

However, the above-mentioned robotic system may not be suitable for surgical types such as hip replacement. Since mentioned above, many operations are required in the hip replacement (such as reaming acetabulum, beating an acetabular cup, and reaming on the side of the femur), and corresponding, surgical tools with different constructions are needed. A system designed to adapt to various tools requires various end effectors, and removal and installation of different types of the actuators to the robotic arm during a surgical procedure will increase surgical time. In addition, the process of beating the acetabular cup to the acetabular fossa will produce a high impact force, which may damage the precision robotic arm.

Therefore, an actuator suitable for hip surgery of robot system is needed.

MAKO SURGICAL CORP. also provides a surgical robot for the hip replacement, and the Chinese invention patent CN102612350B discloses its configuration. When the surgical robot is used to grind the acetabulum, it is necessary to first install a grinding tool to a holding structure on an end of the robotic arm, and then a power apparatus is connected to the grinding tool. This holding structure is also used to connect a cup holder to perform an operation for installing a prosthesis. Therefore, after an operation of acetabulum grinding is completed, it is necessary to first remove a power apparatus, and then remove the grinding tool, and finally install the cup holder to the holding structure. The operations in the above-mentioned process are rather complicated.

### SUMMARY

The present disclosure provides an actuator for hip replacement and a surgical system, so that each surgical stage of the hip joint surgery is capable to be performed more conveniently and smoothly.

In the present disclosure, an actuator for hip replacement is provided, which is configured to prepare a space for installing a prosthesis on a bone and implanting the prosthesis, including a first actuator and a second actuator. The first actuator is configured to connect a cutting tool to process an acetabulum and/or a medullary cavity, and the first actuator has a first connector and a second connector. The second actuator is configured to connect to the second connector of the first actuator during an operation of implantation of the prosthesis is performed, and connect the prosthesis and receive impact (also called shock) produced during installing the prosthesis. Where, the actuator for the hip replacement is configured to be installed to a robotic arm via the first connector.

In the present disclosure, a joint forming actuator is also provided, and the joint forming actuator includes a power apparatus and a tool assembly. The power apparatus includes a robot connecting end and a built-in power assembly. The robot connecting end is configured to connect to an end of a robotic arm of a robot, and the power assembly includes a power source and an output shaft. The output shaft is connected to the power source. The tool assembly includes a connecting part and a surgical tool, the surgical tool is rotatably provided on the connecting part, and the tool assembly is detachably provided on the power apparatus through the connecting part. When the tool assembly is connected to the power apparatus through the connecting part, the surgical tool forms a joint with the output shaft to receive rotation movement output by the output shaft.

In the present disclosure, an actuator for installing a prosthesis is also provided, for performing a surgical plan under the control of a robotic arm. The actuator for installing the prosthesis includes a sliding rod, a supporting assembly and a tracer. A first end of the sliding rod is configured to connect the prosthesis, and a second end of the sliding rod is configured to receive impact produced during installation of the prosthesis. The supporting assembly includes an accommodating channel accommodating a portion of a rod section of the sliding rod. The sliding rod is axially movable with respect to the supporting assembly. The supporting assembly is configured to connect the actuator for installing prosthesis to an end of a robotic arm of a robot, and the tracer is provided on the sliding rod to indicate an orientation of the sliding rod.

In the present disclosure, a tool assembly is also provided, for being connected and driven by a power apparatus to machine a joint. The tool assembly includes a connecting part and a surgical tool, the surgical tool is rotatably provided on the connecting part, and the connecting part is configured to detachably connect the tool assembly to the power apparatus. When the tool assembly is connected to the power apparatus via the connecting part, the surgical tool forming a joint with an output shaft of the power apparatus to receive rotation movement output by the output shaft.

In the present disclosure, a surgical system is provided, including an actuator, a robotic arm, a navigation system and a controlling system. The actuator is the actuator for the hip replacement as described in the first aspect. The robotic arm is connected to the first connector of the actuator. The navigation system is configured to measure a position of the actuator. The controlling system is configured to drive the robotic arm to move the actuator to a target position according to a surgical plan.

The actuator for the hip replacement as provided in the present disclosure includes the first actuator and the second actuator. The first actuator is configured to connect the cutting tool, to process the acetabulum and/or the medullary cavity. The second actuator is connected to the first actuator during the operation of the implantation of the prosthesis is performed, and is configured to connect the prosthesis and receiving the impact produced during installing the prosthesis. During the hip joint surgery, when the acetabulum and the medullary cavity are prepared, the robotic arm is connected to the first actuator. When it is necessary to install the prosthesis, the second actuator is connected to the first actuator. By the above configuration, the operation of actuator replacement is capable to be reduced, thereby facilitating completing the hip replacement surgery.

### BRIEF DESCRIPTION OF THE DRAWINGS

FIG. 1 is a schematic of a surgical system according to an embodiment of the present disclosure.
FIG. 2 is a schematic diagram of an actuator for hip replacement according to an embodiment of the present disclosure.
FIG. 3 is a schematic diagram of usage of a first actuator according to an embodiment of the present disclosure.
FIG. 4 is a structural schematic diagram of a power apparatus according to an embodiment of the present disclosure.
FIG. 5 is a section view of an inner structure of a power apparatus according to an embodiment of the present disclosure.
FIG. 6 is a structural schematic diagram of an output shaft of the power apparatus in FIG. 3 according to an embodiment of the present disclosure.
FIG. 7 is a structural schematic diagram of an output shaft according to an embodiment of the present disclosure.
FIG. 8 is a structural schematic diagram of a coupling according to an embodiment of the present disclosure.
FIG. 9 is a structural schematic diagram I of a connector and an output shaft according to an embodiment of the present disclosure.
FIG. 10 is a section view of structures of a connector and an output shaft according to an embodiment of the present disclosure.
FIG. 11 is a schematic diagram I of a first type tool assembly according to an embodiment of the present disclosure.
FIG. 12 is a section view of a first type tool assembly according to an embodiment of the present disclosure.
FIG. 13 is a structural schematic diagram of a connecting part according to an embodiment of the present disclosure.
FIG. 14 is a connecting schematic diagram of a rotating joint structure and a spline according to an embodiment of the present disclosure.
FIG. 15 is a section view of structures of a power apparatus and a first type tool assembly according to an embodiment of the present disclosure.
FIG. 16 is a structural schematic diagram of a joint between a power apparatus and a first type tool assembly according to an embodiment of the present disclosure.
FIG. 17 is a schematic diagram of another connecting structure of an output shaft and an adapter shaft according to an embodiment of the present disclosure.
FIG. 18 is a schematic diagram of yet another connecting structure of the output shaft and the adapter shaft according to an embodiment of the present disclosure.
FIG. 19 is a structural schematic diagram of a first actuator, which is connected to a second type tool assembly, according to an embodiment of the present disclosure.
FIG. 20 is a schematic diagram of a whole structure of a second actuator according to an embodiment of the present disclosure.
FIG. 21 is a section view of a whole structure of a second actuator according to an embodiment of the present disclosure.
FIG. 22 is a structural schematic diagram of a joint between a supporting assembly and a sliding rod according to an embodiment of the present disclosure.
FIG. 23 is a schematic diagram of components at an accommodating channel according to an embodiment of the present disclosure.
FIG. 24 is a schematic diagram of a second actuator installed by a first actuator according to an embodiment of the present disclosure.
FIG. 25 is a structural schematic diagram I of a supporting assembly and a second connector according to an embodiment of the present disclosure.
FIG. 26 is a structural schematic diagram II of a supporting assembly and a second connector according to an embodiment of the present disclosure.
FIG. 27 is a structural schematic diagram III of a supporting assembly and a second connector according to an embodiment of the present disclosure.
FIG. 28 is a structural schematic diagram of a sliding rod installed with an adjusting member according to an embodiment of the present disclosure.
FIG. 29 is a schematic diagram I of an adjusting member according to an embodiment of the present disclosure.
FIG. 30 is a schematic diagram II of an adjusting member according to an embodiment of the present disclosure.
FIG. 31 is a schematic diagram III of an adjusting member according to an embodiment of the present disclosure.
FIG. 32 is a structural schematic diagram of a nut according to an embodiment of the present disclosure.
FIG. 33 is a structural schematic diagram of a cap nut according to an embodiment of the present disclosure.
FIG. 34 is a structural schematic diagram I of an actuator for installing a prosthesis provided in an embodiment of the present application.
FIG. 35 is a section view of an actuator for installing a prosthesis provided in an embodiment of the present application.
FIG. 36 is a partially section view of an actuator for installing a prosthesis provided in an embodiment of the present application.
FIG. 37 is an enlarged view of a P-region in FIG. 35 provided in an embodiment of the present application.
FIG. 38 is a schematic diagram of components of an actuator for installing a prosthesis provided in an embodiment of the present application.
FIG. 39 is a schematic diagram of components of an actuator for installing a prosthesis provided in an embodiment of the present application.
FIG. 40 is a local schematic diagram of an actuator for installing a prosthesis provided in an embodiment of the present application.
FIG. 41 is a local schematic diagram of an actuator for installing a prosthesis provided in an embodiment of the present application.
FIG. 42 is a local schematic diagram II of an actuator for installing a prosthesis provided in an embodiment of the present application.
FIG. 43 is a three-dimensional structural schematic diagram I of an actuator for installing a prosthesis provided in another embodiment of the present invention.
FIG. 44 is a section view structural schematic diagram of an actuator for installing a prosthesis provided in an embodiment of the present invention.
FIG. 45 is a three-dimensional structural schematic diagram II of an actuator for installing a prosthesis provided in an embodiment of the present invention.
FIG. 46 is a three-dimensional structural schematic diagram III of an actuator for installing a prosthesis provided in an embodiment of the present invention.
FIG. 47 is an exploded view of a connection state of a cover body and a sliding rod of an actuator for installing a prosthesis provided in an embodiment of the present invention.
FIG. 48 is a schematic diagram of a connection state of a cover body and a sleeve of an actuator for installing a prosthesis provided in an embodiment of the present invention.

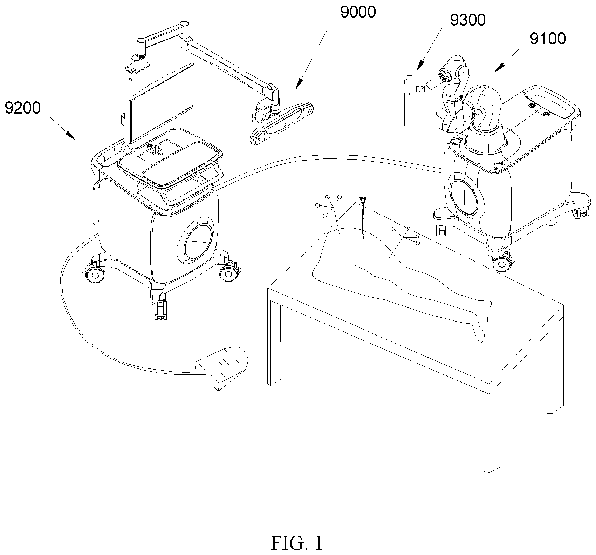

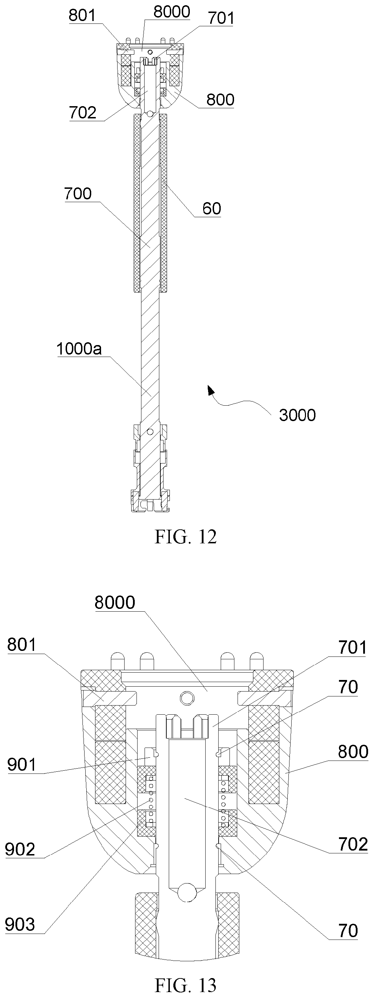

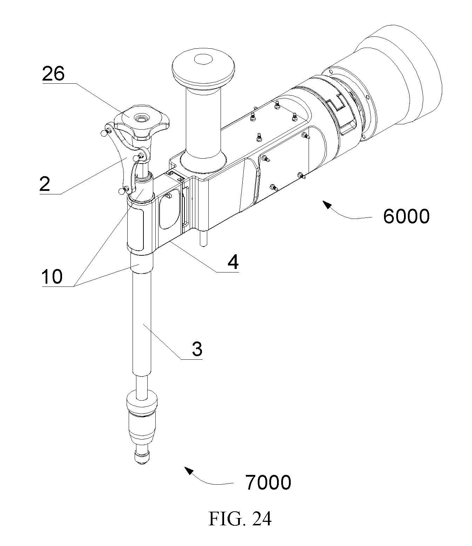

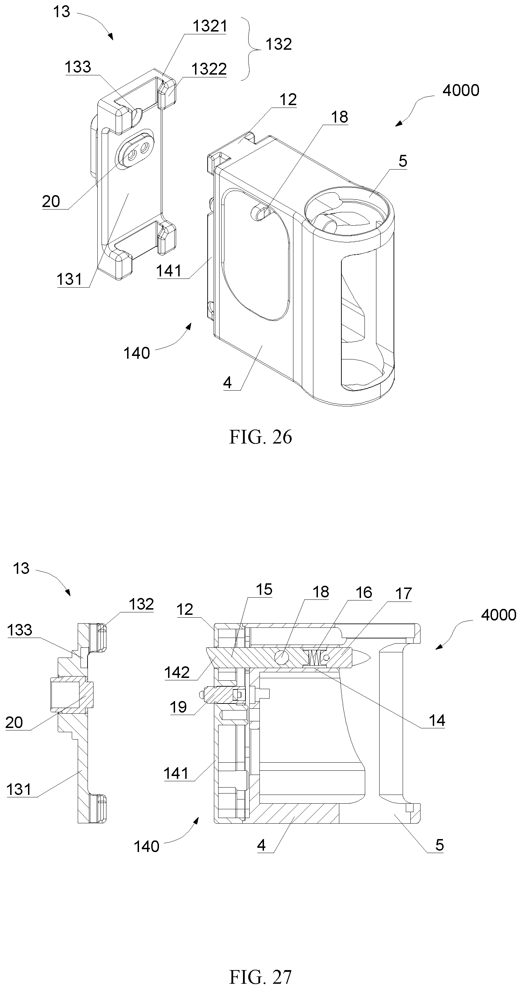

### Description of reference numerals of FIG. 1 to FIG. 33:

1-sliding rod, 2-tracer, 3-holding part, 4-supporting member, 5-accommodating channel, 6-insulating sleeve, 7-sliding sleeve, 8-first buffering member, 9-check ring, 10-insulating member, 11-second buffering member, 12-inserting block, 13-second connector, 14-installing hole, 15-locking pin, 16-first elastic member, 17-spacer block, 18-locking pin pulling bolt, 19-identifying plug, 20-identifying base, 21-adapter shaft, 22-nut, 23-reducing sleeve, 24-spline, 25-holding member, 26-cap nut, 27-adjusting member, 28-first position, 29-second position, 30-first connector, 40-handle, 50-insulating cover, 60-holding sleeve, 70-ring groove, 80-axial buffering mechanism, 90-axial limit structure;
100-housing, 101-check rim, 121-limit groove, 131-bottom plate, 132-limit button, 1321-first section, 1322- second section, 133-locking pin hole, 140-quick release mechanism, 141-first limit mechanism, 142-second limit mechanism, 150-tracing assembly, 151-tracing element;
200-motor, 210-main shaft section, 211-connecting hole, 212-clamping block, 213-flange, 214-limit section, 215-limit step, 221-outer wall, 222-clamping groove, 223-spline keyway, 261- force-bearing plate, 262-connecting section;
300-speed reducer;
400-output shaft, 401-input section, 402-middle section, 403-output section, 4031-coupling spline, 404-positioning hole; 500-coupling, 501-first portion, 502-second portion;
600-joint, 601-hole, 602-spiral groove, 6020-limiting part, 6021-rotating section, 6022-positioning section, 603-hole; 610-rotating joint structure;
700-connecting rod main shaft, 701-spline joint, 702-joint hole, 703-positioning shaft, 710-spline connection, 720-radial positioning structure;
800-connecting rod lock head, 801-positioning pin;
900-positioning module, 901-clamping support, 902-elastic member, 903-sliding sleeve;
1000a-surgical tool, 1000b-surgical tool, 1001-reamer rod, 1002-reamer blade, 1003-prosthesis, 1004-cutting tool;
2000-power apparatus, 2100-power assembly, 2200-power source, 3000-tool assembly, 4000-supporting assembly, 5000-adjusting assembly, 6000-first actuator, 7000-second actuator, 8000-connecting part, 9000-navigation system, 9100-robotic arm, 9200-controlling system, 9300-actuator for hip replacement.

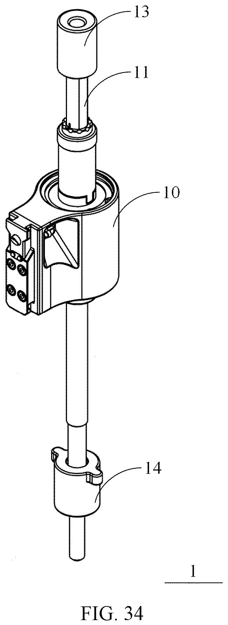

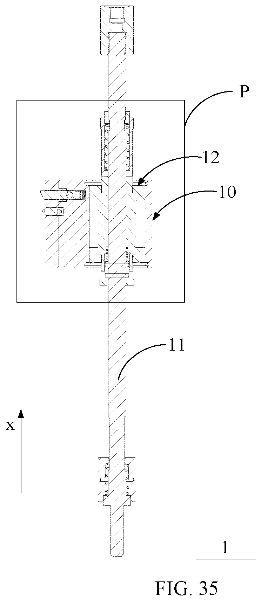

### Description of reference numerals of FIG. 34 to FIG. 42:

1-actuator for installing prosthesis; 10-supporting assembly; 101-accommodating channel; 103-locking pin spring; 104-locking pin; 105-contact; 106-connecting block; 107-snap spring pad groove; 108-snap spring groove; 11-sliding rod; 111-third groove; 1111-first portion; 1112-second portion; B1-plane part; B2- arc-surfaced part; 112-fourth groove; 113-first via hole; 114-fifth groove; 12-first damping mechanism; 121-sleeve; 122-first elastic member; A1-first end; A2-second end; A3-middle region; L-preset gap; 13-hitting cap; 14-acetabular cup connecting mechanism; 15-first circumferential direction limit structure; 151-first groove; 152-first protrusion; 16-second circumferential direction limit structure; 161-second groove; 162-second protrusion; 17-second damping mechanism; 171-first limit block; 1711-second clamping end; 172-first pressure spring; 1721-first clamping end; 173-pressure spring cover; 1731-third clamping end; 18-third damping mechanism; 181-second limit block; 1811-second via hole; 182-limit pin; 183-second pressure spring; 19-axial limit structure; 191-snap spring; 192-snap spring pad; x-length direction.

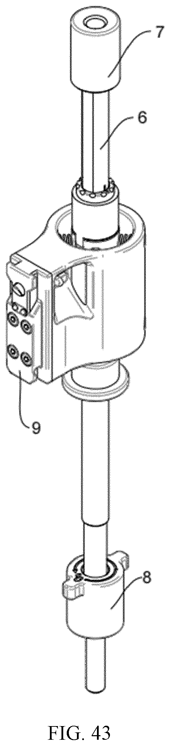

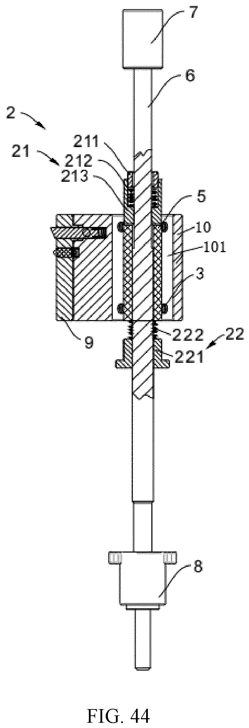

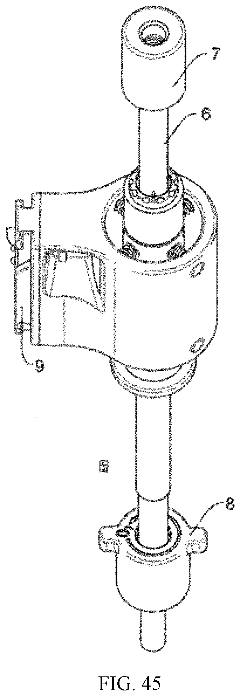

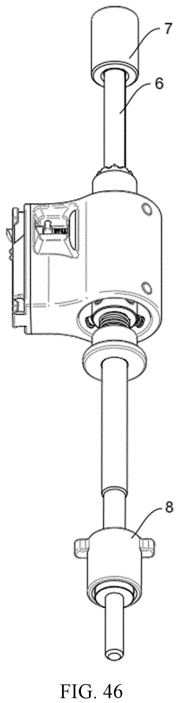

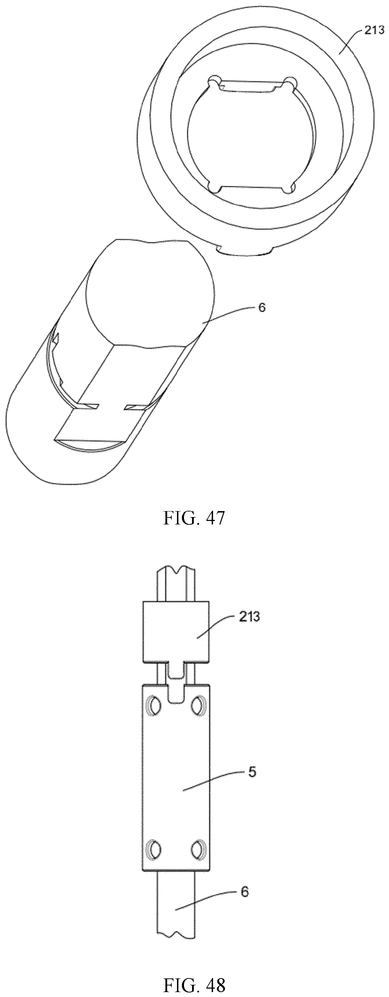

### Description of reference numerals of FIG. 43 to FIG. 48:

1-robotic arm; 2-axial buffering mechanism; 21-first buffering mechanism; 211; first limit block; 212-first elastic structure; 213-cover; 22-second buffering mechanism; 221-second limit block; 222-second elastic structure; 3-buffering assembly; 4-controlling system; 5-sleeve; 6-sliding rod; 7-hitting cap; 8-acetabular cup connecting apparatus; 9-connecting mechanism, 10-supporting assembly, 101-accommodating channel.

### DETAILED DESCRIPTION OF THE EMBODIMENTS

Hereinafter, features and exemplary embodiments of various aspects of the present disclosure will be described in detail. In order to make purposes, technical solutions and advantages of the present disclosure clearer and more understandable, the present disclosure will be further described in detail in combination with the drawings and specific embodiments as below. It should be understood that, the specific embodiments as described herein are intended merely to explain the present disclosure, rather than limiting the present disclosure. For a person skilled in the art, the present disclosure may be implemented without some of the specific details. The description for the embodiments as below is intended merely to provide better understanding to the present disclosure by illustrating examples of the present disclosure.

It should be noted that, in the present specification, the relation terms, such as "first" and "second" or the like, are used only to distinguish one entity or operation from another entity or operation, rather than requiring or implying that there is any actual relationship or order between these entities or operations. Moreover, the terms "comprise", "include" or any other variations thereof is intended to cover non-exclusive inclusion, such that any process, method, item or device including a series of elements not only includes said elements, but also further includes other element(s) not specifically listed, or further includes the element(s) inherent to the process, method, item or device. In the cases without any more limitation(s), the element(s) as defined by a sentence of "include......" will not exclude the case(s) in which the process, method, item or device including said elements further includes additional same element(s).

It should be noted that, the embodiments and the features therein in the present disclosure may be combined with one another, unless it causes contradiction. Hereinafter, the embodiments will be described in detail in combination with the drawings.

As shown in FIG. 1, a robotic system as provided in the present disclosure includes a robotic arm 9100, a navigation system 9000, an actuator for hip replacement 9300 and a controlling system 9200. The robotic arm 9100 corresponds to an arm of a surgeon, and it may hold a surgical tool and position the surgical tool in a relatively high precision. The navigation system 9000 corresponds to an eye of the surgeon, and it may measure a position of the surgical tool and a tissue of a patient in real time. The controlling system 9200 corresponds to a brain of the surgeon, and it stores a surgical plan therein. The controlling system 9200 calculates a path for the robotic arm and/or its position to be reached according to information obtained by the navigation system 9000 during surgery, and may actively control movement of the robotic arm 9100, or configure a virtual boundary for the robotic arm 9100 through a force feedback mode, and then manually push the robotic arm 9100 to move along a path or face as defined by the virtual boundary or within a defined volume.

The actuator for the hip replacement 9300 is configured to prepare a space for installing a prosthesis on a bone and implanting the prosthesis. The actuator for the hip replacement includes a first actuator and a second actuator. The first actuator is configured to connect a cutting tool to process an acetabulum and/or a medullary cavity. The first actuator has a first connector and a second connector. The second actuator is configured to connect to the second connector of the first actuator during an operation of implantation of the prosthesis is performed, and for connecting the prosthesis and receiving impact produced during installing the prosthesis. The actuator for the hip replacement is configured to be installed to a robotic arm 9100 via the first connector. In a hip joint surgery, when it is to process the acetabulum and the medullary cavity, the first actuator is connected onto the robotic arm 9100; and when it is to install the prosthesis, the second actuator is connected to the first actuator. By the above configuration, the operation of replacing the actuator is capable to be reduced.

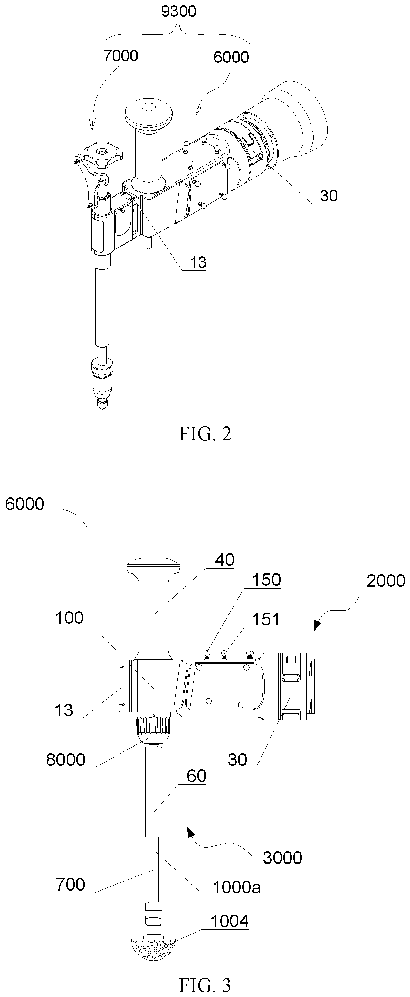

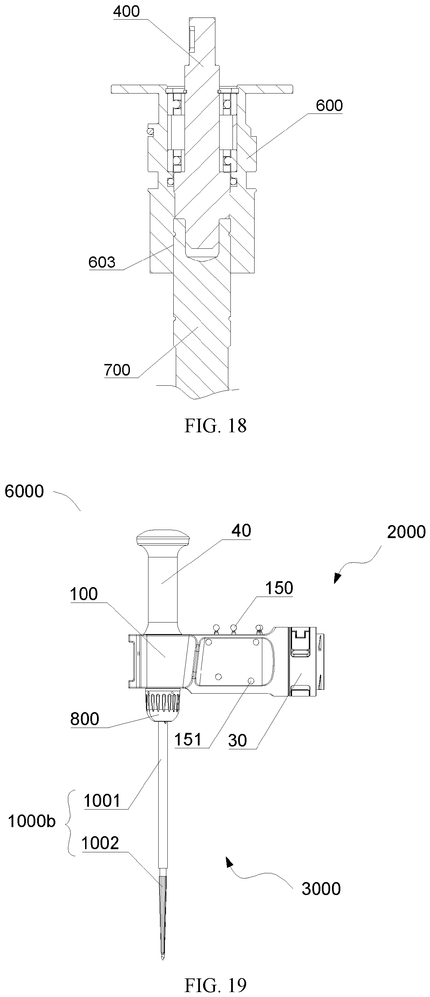

Specifically, in the hip replacement, after a hip joint at an affected area is exposed, it is generally to firstly prepare an acetabular fossa. During this process, it is necessary to use a rotating acetabulum file to grind the acetabular fossa at the affected area, to prepare a shape suitable for installing the prosthesis. FIG. 1 shows a schematic diagram of usage of the first actuator for preparing the acetabular fossa. The first actuator 6000 is connected to the robotic arm 9100 through the first connector 30, the first actuator 6000 is detachably connected to an acetabulum file tool assembly (for example a surgical tool 1000a), and an end of the acetabulum file tool assembly is configured to connect a file head (for example a cutting tool 1004). In this case, the acetabulum file tool assembly may be driven by the first actuator 6000 to grind the acetabulum. After the acetabulum is prepared, it is necessary to place the prosthesis into the acetabulum. FIG. 2 shows a state in which the second actuator 7000 is connected to the second connector 13 of the first actuator 6000 (at this time, the acetabulum file tool assembly on the first actuator 6000, used for grinding the acetabulum, is removed). The second actuator 7000 is connected to the robotic arm 9100 indirectly through the first actuator 6000, and the prosthesis may be installed, under holding of the robotic arm 9100. Further, as shown in FIG. 18, if it is to perform reaming on a proximal end of a femur, it is necessary to remove the second actuator 7000 from the second connector 13, and install a medullary cavity reamer assembly (for example a surgical tool 1000b) for reaming onto the first actuator 6000.

Hereinafter, the first actuator 6000 will be described. A tool assembly 3000 therein is a connecting rod for connecting the acetabulum file head, as shown in FIGs, 1 to 19.

The first actuator 6000 is a joint forming actuator, which is configured to prepare the formed acetabular fossa or medullary cavity on a hip joint. The first actuator 6000 includes a power apparatus 2000 and a tool assembly 3000. The power apparatus 2000 includes a housing 100 and a built-in power assembly 2100. The first actuator 6000 is connected to an end of the robotic arm 9100 of the robot. The power assembly 2100 includes a power source 2200 and an output shaft 400, and the output shaft 400 is connected to the power source 2200. The tool assembly 3000 includes a connecting part 8000 and a surgical tool 1000a, and the surgical tool 1000a is rotatably provided on the connecting part 8000. The tool assembly 3000 is detachably provided on the power apparatus 2000 through the connecting part 8000. When the tool assembly 3000 is connected to the power apparatus 2000 via the connecting part 8000, the surgical tool 1000a is joined the output shaft 400 to receive rotation movement output by the output shaft 400. The power assembly 2100 is provided in the housing 100 and outputs power through the output shaft 400. The output shaft 400 is engaged with an end of the tool assembly 3000 to drive a connecting rod of the grinding file, without using a long guiding cylinder to guide the connecting rod, so that a structure of the actuator is more compact. In this way, interference and influence on a surgery space and influence on safety due to an externally connected power source is reduced, and operations of assembling the externally connected power source during surgery is reduced, and thus the surgery process is smoother.

Specifically, as shown in FIG. 2 and FIG. 4 to FIG. 6, the first actuator 6000 includes a power apparatus 2000 and a tool assembly 3000. The power apparatus 2000 includes a housing 100 and a power assembly 2100. The housing 100 is an internal hollow part with a generally quadrangular shape. The housing 100 is provided, at its two ends, with a first connector 30 and a second connector 13, respectively. The first connector 30 is used as a connecting end to connect the first actuator 6000 to the robotic arm 9100. The second connector 13 is used as a connector for an actuator for installing a prosthesis, and is used for detachably connecting the actuator for installing the prosthesis (for example a second actuator 7000). The housing 100 is further provided thereon with a handle 40. The handle 40 is hollow inside, and is detachably connected to the housing 100. A structure of the power apparatus 2000 used for connecting the tool assembly 3000 is a quick joining joint, which is provided on another side of the housing 100 opposite to the position of the handle 40. When the tool assembly 3000 is installed to the quick joining joint, an axis of the handle 40 and an axis of an acetabulum file rod assembly are basically in a straight line, and they are arranged on two sides of the power apparatus 2000. Each surface of the housing 100 is used for connecting a tracing assembly 150 to indicate a position of the actuator.

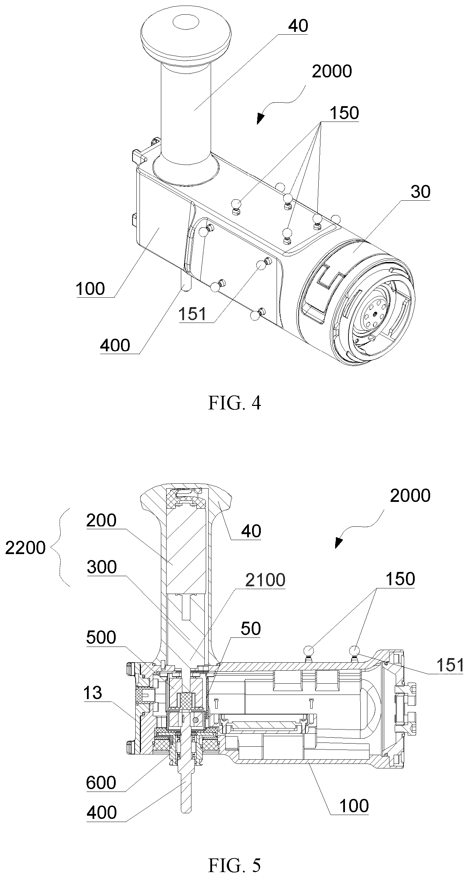

As shown in FIG. 5, the power assembly 2100 includes a motor 200, a speed reducer 300, an output shaft 400 and a coupling 500. The motor 200 and the speed reducer 300 constitute the power source 2200. The power source 2200 is integrated in the handle 40, and is fixedly connected to the housing 100. The speed reducer 300 includes a shaft connected to the output shaft 400 via the coupling 500. The power source 2200 and the output shaft 400 are provided coaxially, with the axes thereof perpendicular to the housing 100.

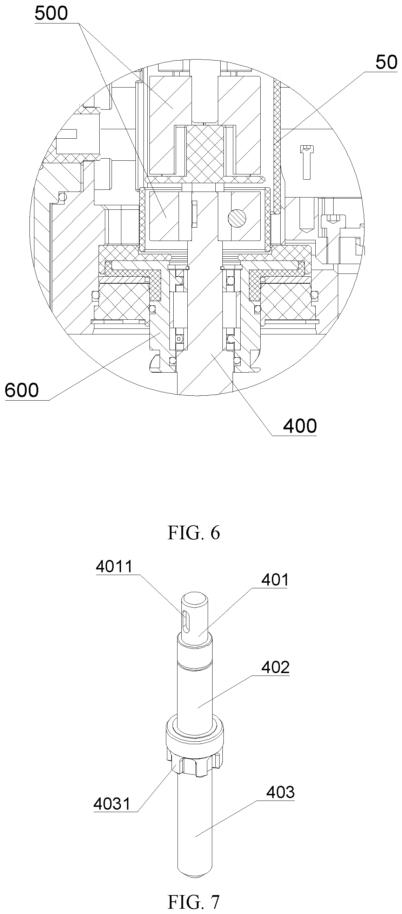

As shown in FIG. 7, the output shaft 400 includes an input section 401, a middle section 402 and an output section 403 which are provided in sequence. The input section 401 is provided thereon with a keyway 4011 for receiving rotation movement from the power source 2200. The middle section 402 is installed in a bearing in the power apparatus 2000. The output section 403 is provided with a coupling spline 4031 which includes a plurality of protrusions distributed at intervals in a circumferential direction., and is configured to output a torque. A length of the coupling spline 4031 is less than a length of the output section 403. That is, the last section of the output section 403 is a smooth shaft.

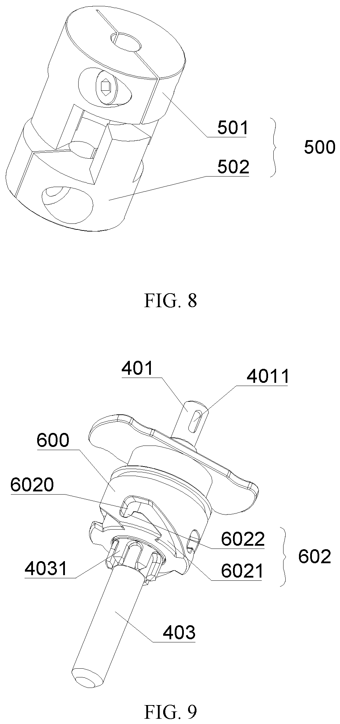

As shown in FIG. 8, the coupling 500 is a plum coupling. The coupling includes a first portion 501 and a second portion 502, and both the first portion 501 and the second part 502 are provided with locking screws for fixing a shaft, and an insulating sleeve is provided between the first portion 501 and the second portion 502. A shaft at an output end of the speed reducer 300 is connected to the first portion 501 via a coupling spline and the locking screws, and the output shaft 400 is also connected to the second portion 502 through the key connection and the locking screws. The key connection between the coupling 500 and the shaft at the output end of the speed reducer 300, as well as the key connection between the coupling 500 and the output shaft 400, on one hand, increase reliability of the transmission based on the locking screws, and on the other hand, the key connection improves the maximum torque that may be transmitted.

Referring to FIG. 5 and FIG. 6, inside the first actuator 6000, the outer periphery of the coupling 500 is provided with an insulating cover 50. The insulating cover 50 may isolate the housing 100 from the speed reducer 300, to prevent electric leakage of the motor 200 from being conducted to the housing 100 through the speed reducer 300. The insulating cover 50 has a function of isolating electrical wires/wires, and prevents the electrical wires/wires inside the housing 100 from rubbing or winding with the rotating coupling 500.

Referring to FIG. 5 to FIG. 7, FIG. 9 and FIG. 10. herewith, the housing 100 is further provided thereon with a joint 600 fixed to the housing 100.

The joint 600 is used for connecting the tool assembly 3000 and installing the output shaft 400. The joint 600 has a columnar shape, and is provided therein with a hole 601 and is provided with four spiral grooves 602 on its periphery. The spiral groove 602 is used for guiding a pin shaft member and includes a limiting part 6020 for limiting an axial direction and a circumferential direction of the pin shaft member. An end of the joint 600 is provided with two wing plates along a radial direction. The hole 601 is used for installing a bearing therein and accommodating the middle section 402 of the output shaft 400. The spiral groove 602 includes a rotating section 6021 and a positioning section 6022. The rotating section 6021 spirally extends in a first axial direction, the positioning section 6022 extends from an extending end of the rotating section 6021 towards a second axial direction, and the second axial direction is opposite in direction to the first axial direction. A side wall of the positioning section 6022 forms the limit portion 6020. The positioning section 6022 is used to form limit in the second axial direction and limit in a circumferential direction to an object contained in the groove. The wing plate is used for fixing the joint 600 with the housing 100. When the output shaft 400 is installed to the joint 600, the coupling spline 4031 extends out of the hole 601 and is located outside the housing 100.

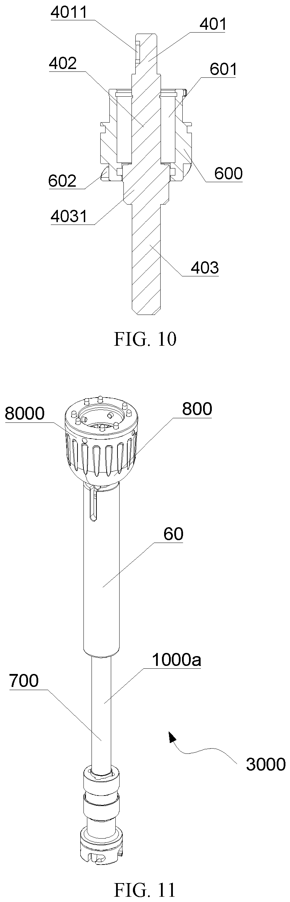

As shown in FIG. 11 to FIG. 13, the tool assembly 3000 includes a connecting part 8000 and a surgical tool 1000a. The surgical tool 1000a is rotatably provided, by its one end, on the connecting part 8000. The surgical tool 1000a is an acetabulum grinding file rod assembly, and is connected, by its other end, with the acetabulum grinding file. The acetabulum grinding file rod assembly includes a connecting rod main shaft 700, an acetabulum file connecting component and a holding sleeve 60. An end of the connecting rod main shaft 700 is rotatably connected to the connecting part 8000, and the other end of the connecting rod main shaft 700 is provided with the acetabulum file connecting component. The holding sleeve 60 is sleeved outside the connecting rod main shaft 700. An end of the connecting rod main shaft 700 connected to the connecting part 8000 is provided with a spline joint 701and an joint hole 702. The spline joint 701 is capable to be engaged and matched with the coupling spline 4031, to achieve transmission of the rotation movement. But they are not closely fitted, and may be separated in an axial direction. A diameter of the joint hole 702 is equal to a diameter of the smooth shaft portion of the output section 403.

The connecting part 8000 includes a connecting rod lock head 800 and a connecting rod connection module. The connecting rod lock head 800 has an internal hollow cup shape, and is provided with a circular hole on its bottom. The connecting rod lock head 800 is provided, in positions on its inner circumferential face close to an opening, with four positioning pins 801 distributed circumferentially. The connecting rod connection module is provided in the connecting rod lock head 800, and is used for rotatably connecting the acetabulum grinding file rod assembly to the connecting rod lock head 800.

The connecting rod connection module includes a clamping support 901, a positioning module 900 and a pair of sliding sleeves 903, which are coaxially held in the connecting rod lock head 800. The clamping support 901 is in a ring shape and is provided on the outermost side (a side where the opening of the connecting rod lock head 800 is located). The positioning module 900 includes an elastic member 902, and is configured to form a predetermined acting force between the connecting part 8000 and the power apparatus 2000. In the present embodiment, the elastic member 902 is a thrust spring. Both the two sliding sleeves 903 are in a ring shape, and are provided axially between the clamping support 901 and a bottom of the connecting rod lock head 800. An outer circumferential face of the sliding sleeve 903 is fitted with an inner circumferential face of the connecting rod lock head 800, and a diameter of an inner hole of the sliding sleeve 903 is equal to a diameter of the connecting rod main shaft 700. The thrust spring is provided between the two sliding sleeves 903.

The connecting rod main shaft 700 is sleeved in the clamping support 901, the thrust spring and the sliding sleeves 903. A peripheral face of the connecting rod main shaft 700 is further provided with two ring grooves 70 having a predetermined distance, and the ring grooves 70 are used for installing baffle rings. With the assembling relation, the clamping support 901, the thrust spring, the sliding sleeves 903 and the connecting rod lock head 800 are all located between the two baffle rings. Therefore, the connecting rod lock head 800 and the connecting rod main shaft 700 form an integrated structure. The thrust spring may be compressed, thus allowing a certain amount of movement of the connecting rod lock head 800 in an axial direction of the connecting rod main shaft 700.

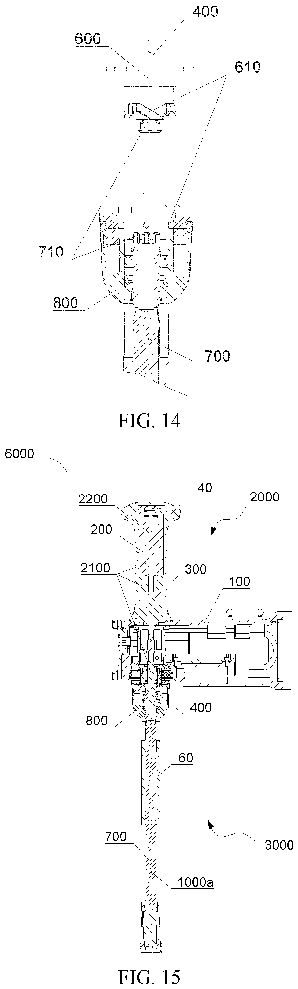

As shown in FIG. 14, the connecting part and the power apparatus 2000 will be connected by a rotating joint structure 610 to form an axial and circumferential direction limit to the connecting part. Herein, the rotating joint structure 610 includes a positioning pin 801 and a spiral groove 602. That is, the tool assembly 3000 is connected to the housing 100 through the positioning pin 801 being rotating joint and fitting with the spiral groove 602.

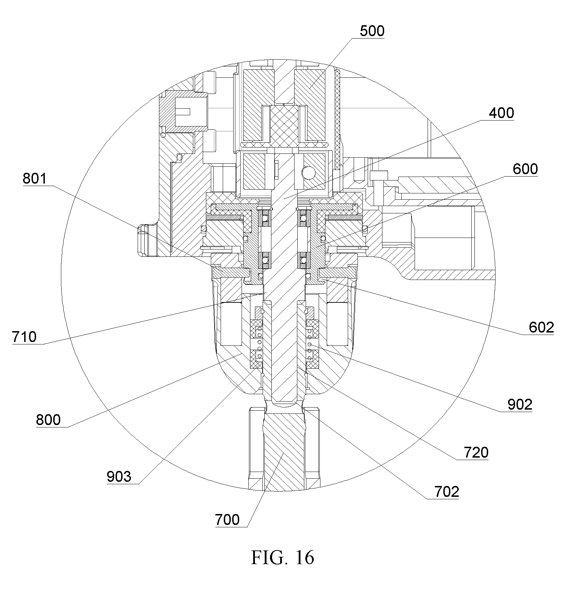

FIG. 15 and FIG. 16 show structural schematic diagrams of an acetabulum grinding file rod assembly installed to the power apparatus 2000. With the assembling relation, the positioning pin 801 is inserted and connected in the positioning section 6022 of the spiral groove 602. Two side walls of the positioning section 6022 extending in an axial direction form the circumferential direction limit to the positioning pin 801, and an end wall of the positioning section 6022c forms the axial limit to the positioning pin 801. Therefore, if there is no external force applied, the connecting rod lock head 800 will not drop axially or rotate circumferentially. The connecting part 8000 forms radial positioning with the connecting rod main shaft 700 and the housing 100, which is equivalent to that the radial positioning is formed between the connecting rod main shaft 700 and the output shaft 400 (which is positioned on the housing 100). Specifically, referring to FIG. 14 and FIG. 16, the smooth shaft portion of the output shaft 400 and the joint hole 702 of the connecting rod main shaft 700 form a radially positioning structure 710. The radially positioning structure 710 is a shaft-hole fit structure, with equal diameters. That is, a direct radial positioning is formed between the output shaft 400 and the joint hole 702. With limitation to a length and a fitting precision of a fitting section for forming the radial positioning between the connecting part 8000 and the connecting rod main shaft 700, a certain amount of radial movement of the connecting rod main shaft 700 may be possible. The radial positioning between the smooth shaft portion of the output shaft 400 and the joint hole 702 of the connecting rod main shaft 700 is capable to improve precision of the radial positioning. The spline joint 701 of the connecting rod main shaft 700 is aligned and engaged with the coupling spline 4031 of the output shaft 400 to receive the rotation movement. The axial acting force applied by the thrust spring to the connecting rod lock head 800 causes the positioning pin 801 to be pressed axially against the end wall of the positioning section 6022. As the thrust spring is compressed, there is an internal stress in the connection between the connecting part 8000 and the power apparatus 2000. Such internal stress enables stable axial positioning to be formed between the tool assembly 3000 and the power source, without increasing difficulty of design or difficulty of installation to ensure the precision of the axial positioning, and thus provides more stable connection and avoids loosening due to vibration or the like. Moreover, the connecting rod main shaft 700 is pushed axially by the thrust spring against the output shaft 400 to form axial positioning.

Compared with thread screwing connection, the cooperation of the positioning pin 801 and the spiral groove 602 is labor-saving, facilitating fast assembling/disassembling during surgery. The direct physical limit of the positioning section 6022 to the positioning pin 801 is more reliable than frictional locking. In some optional embodiments, the positioning pin 801 may be provided on the outer circumferential face of the connecting rod lock head 800, and the spiral groove 602 may be provided on the inner circumferential face of the joint 600. In other optional embodiments, the positioning pin 801 may be provided on the inner/outer circumferential face of the joint 600, and the spiral groove 602 may be provided on the outer/inner circumferential face of the connecting rod lock head 800. By such configuration, it also ensures that the positioning pin 801 and the spiral groove 602 are capable to be rotated and joined when they cooperate with each other, and further the axial and circumferential positioning of the joint 600 and the connecting rod lock head 800 is enabled.

The joint between the output shaft 400 and the connecting rod main shaft 700 is achieved by a spline connection 710 in which during joining, it is only necessary to make the connecting rod main shaft 700 align the output shaft 400 axially, convenient in operation. In some optional embodiments, it is also possible to form connection allowing torque transmission between the output shaft 400 and the connecting rod main shaft 700 by mutual embedding of end faces.

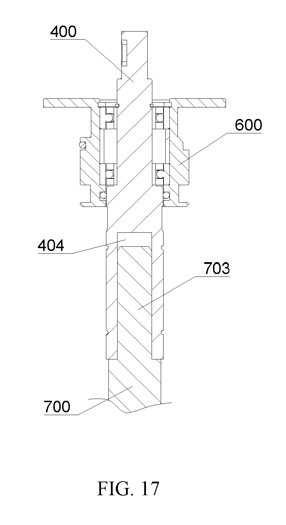

As shown in FIG. 17, in some optional embodiments, it is possible to use another alternative radially positioning structure for the radial positioning between the smooth shaft portion of the output shaft 400 and the joint hole 702 of the connecting rod main shaft 700. For example, a positioning shaft 703 is provided at an end of the connecting rod main shaft 700, and a positioning hole 404 is provided on the output shaft 400, thus forming the radial positioning by the shaft-hole fit thereof. Or, as shown in FIG. 18, a shaft-hole fit structure is provided between the connection joint 600 and the connecting rod main shaft 700. For example, a hole 603, having a diameter larger than a diameter of the spline portion of the output shaft 400, is provided at an end of the joint 600, and accordingly an end of the connecting rod main shaft 700 is configured to have the same diameter, thus forming the shaft-hole fit therebetween.

In some optional embodiments, it is also possible to provide a spring in other positions, functioning as the elastic member 902 in the positioning module 900, to form the internal stress between the tool assembly 3000 and the power apparatus 2000. For example, a pressure spring is fixed on the power apparatus 2000. When the tool assembly 3000 is installed to the power apparatus 2000, the connecting rod lock head 800 compresses the pressure spring, and the reaction force of the pressure spring tightly presses the positioning pin 801 of the connecting rod lock head 800 in the spiral groove 602, such that a pre-pressure is maintained between the connecting rod lock head 800 and the power apparatus 2000, forming relatively stable connection. In a state of final use, the connecting rod main shaft 700 will be applied with a reaction force by tissue of a patient to be axially pressed against the output shaft. The pressure spring may include general a coil spring, a disc spring, a wave spring and the like. Certainly, the elastic member 902 is not limited to the form of springs, and it may be an elastic piece.

Hereinafter, the use process of the hip joint former (that is, the first actuator 6000) will be specifically explained.

In use, the first actuator 6000 is connected, via the first connector 30, with the robotic arm 9100, and at this time, the first actuator 6000 is not installed with the tool assembly 3000. Firstly, the robotic arm 9100 comes into the ready position according to the predetermined surgical plan. A surgeon installs the acetabulum grinding file rod assembly, provided with the acetabulum file (for example the cutting tool 1004), to the first actuator 6000 via the joint 600. Specifically, the surgeon holds the connecting rod lock head 800 in hand, axially sleeves the joint hole of the connecting rod main shaft 700 to the output section 403 of the output shaft 400, and makes the coupling spline 4031 be aligned and joined with the spline joint 701. After the output shaft 400 is circumferentially joined with the connecting rod main shaft 700, the connecting rod main shaft 700 is connected against the output shaft 400. The surgeon pulls and rotates the connecting rod lock head 800 in a direction close to the actuator, such that the positioning pin 801 of the connecting rod lock head 800 moves in the spiral groove 602 along the rotating section 6021 and finally into the positioning section 6022.

Thus, the joint of the coupling spline 4031 and the spline joint 701 results in the circumferential joint of the output shaft 400 and the connecting rod main shaft 700, and the cooperation of the output section 403 and the joint hole 702 improves coaxiality of the connection, thus, together with the connecting rod lock head 800, increasing a length of the radial positioning to the connecting rod main shaft 700, and thus improving the coaxiality of the output shaft 400 and the connecting rod main shaft 700 during transmission of rotation. When the positioning pin 801 is in the positioning section 6022, the positioning pin 801 cannot circumferentially rotate with respect to the joint 600 due to limitation by the two side walls of the positioning section 6022 extending axially. The thrust spring is configured such that there is a trend for the connecting rod lock head 800 to move towards the connecting rod main shaft 700 with respect to the joint 600. Such moving trend prevents the positioning pin 801 from moving axially out of the positioning section 6022 to reach the rotating section 6021. The thrust spring makes the connecting rod main shaft 700 press axially against the output shaft 400. That is, the thrust spring pushes the connecting rod main shaft 700 to keep joining the output shaft 400 axially. In the above operation process, the radially positioning portion of the connecting rod main shaft 700 is its top end. The acetabulum grinding file rod assembly moving axially has a relatively small stroke, and thus the needed operation space is accordingly relatively small.

By now, the acetabulum grinding file rod assembly is connected to the housing 100. With the guidance of the predetermined surgical plan, the first actuator 6000, under control of the robotic arm 9100 and the surgeon, is moved to the predetermined target position. The motor 200 is started and the rotation thereof is transmitted in sequence to the speed reducer 300, the coupling 500 and the output shaft 400. As the output shaft 400 and the connecting rod main shaft 700 are connected via the coupling spline 4031 and the spline connection joint 701, the connecting rod main shaft 700 is driven by the output shaft 400 to rotate. In the process of such rotation, as the connecting rod lock head 800 and the joint 600 are fixedly connected, the connecting rod lock head 800 will not rotate. The rotating connecting rod main shaft 700 drives the acetabulum file (the cutting tool 1004) to rotate, to perform grinding and forming of the acetabular fossa.

After grinding forming of the acetabular fossa is completed according to the predetermined surgical plan, the robotic arm 9100 comes into a posture by which the acetabulum grinding file rod assembly may be disassembled. The surgeon overcomes the elastic force of the thrust spring and pulls the connecting rod lock head 800, such that the positioning pin 801 moves out of the limitation by the positioning section 6022, then rotates the connecting rod lock head 800, and the positioning pin 801 passes the rotating section 6021 and then is detached from the spiral groove 602, and thus the connecting rod lock head 800 is detached from the joint 600. The acetabulum grinding file rod assembly is moved away from the joint 600 in the axial direction of the connecting rod main shaft 700, thus completing disassembling.

In sum of above, the motor 200, the speed reducer 300, the coupling and the output shaft 400 are integrated in the housing 100. A power line of the motor 200 may be introduced by a connector between the housing 100 and the robotic arm 9100. The first actuator 6000 has a compact structure, without providing an external connected power source, and thus avoiding the intervention and influence of the externally connected power source and its power line on the surgery space as well as the safety risk due to exposure of the power line. As it is not necessary to assemble an externally connected power source during surgery, the operation steps of the surgery are reduced. The tool assembly 3000 includes a connecting part 8000 and the acetabulum grinding file rod assembly, and as a pre-assembled modular part, it may be used to achieve removable connection between the surgical tool 1000a and the output shaft 400 conveniently.

As shown in FIG. 19, in an optional embodiment, the surgical tool 1000b is a medullary cavity reamer, and the tool assembly 3000 includes the connecting part 8000 and the medullary cavity reamer. The medullary cavity reamer includes a reamer rod 1001 and a reamer, the reamer is used for reaming and is connected to the reamer rod 1001. The reamer rod 1001 is provided at its end with the spline joint 701, the spline joint 701 is used for connecting with the coupling spline 4031. The reamer is provided thereon with a reamer blade1002, for performing reaming on a medullary cavity of a femur under rotation movement. The connecting part 8000 is same in structure as the above-described connecting part 8000 connected to the acetabulum grinding file rod assembly. The connecting rod connection module connects the reamer rod 1001 with the connecting rod lock head 800. Moreover, the tool assembly 3000 connected to the medullary cavity reamer is also connected to the joint 600 and the output shaft 400 in the same way. After the connecting rod lock head 800 is connected to the joint 600, the medullary cavity reamer uses the spline key connection joint 701 to join the coupling spline key 4031, and thus to the output shaft 400. The output shaft 400 is driven by the motor 200 to drive the medullary cavity reamer to rotate and perform the task of reaming at the proximal end of the femur.

In an optional embodiment, the first actuator 6000 is provided with three groups of tracing assemblies 150. The three groups of tracing assemblies 150 are provided on three surfaces of the housing 100, respectively. Each group includes four tracing elements 151 located on the same plane. As shown in FIG. 2 to FIG. 4, the housing 100 is provided thereon with three planes, and the three groups of the tracing elements 151 are provided on the three planes, respectively. The tracing element 151 may be a passive reflective ball or reflective sheet, or may be an active electromagnetic generator or sensor.

It is understandable that, during a hip joint forming surgery, the tracing element 150 sends position information of the first actuator 6000 to a positioner. The positioner, generally in a constant position in the surgery space, is an apparatus in the navigation system 9000 for receiving position information. By providing three groups of the tracing elements 151, the position information of the first actuator 6000, even in various postures, is capable to be identified. Corresponding to the tracing elements 151, the positioner may be an optical navigator for identifying reflected light, or may be a receiver for identifying an electromagnetic signal.

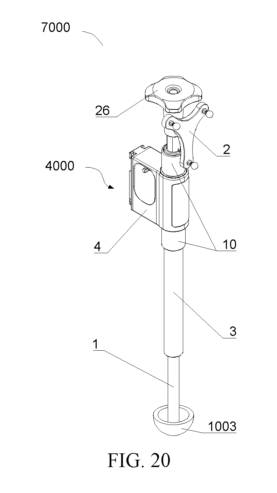

Hereinafter, the second actuator 7000 will be specifically described, as shown in FIG. 20 to FIG. 33.

The second actuator 7000 is an actuator for installing a prosthesis, for installing a prosthesis 1003 during a hip replacement surgery. The actuator for installing the prosthesis includes a sliding rod 1, a supporting assembly 4000 and a tracer 2. A first end of the sliding rod 1 is used for connecting the prosthesis 1003, and a second end of the sliding rod 1 is configured to receive the impact produced during installation of the prosthesis. The supporting assembly 4000 includes an accommodating channel 5 accommodating a portion of a rod section of the sliding rod 1, and the sliding rod 1 is axially movable with respect to the supporting assembly 4000. The supporting assembly 4000 is used for connecting the second actuator 7000 to the robotic arm 9100 of the robot system. The tracer 2 is provided on the sliding rod 1 to indicate the orientation of the sliding rod 1. In the second actuator 7000, the sliding rod 1 is axially movable with respect to the supporting assembly 4000. In use, an axial gap between the sliding rod 1 and the supporting assembly 4000 may be made larger than a stroke of the sliding rod 1 when the sliding rod 1 is hit, to prevent the sliding rod 1 from colliding with the supporting assembly 4000 and damaging the robotic arm 9100 connected to the actuator. The sliding rod 1 and the supporting assembly 4000 are configured as an integrated structure. When the actuator is to be used, it is not necessary to assemble or disassemble the sliding rod 1 and the supporting assembly 4000, and it is only necessary to use the supporting assembly to connect the whole actuator to the robotic arm 9100 or remove it from the robotic arm 9100.

Specifically, in embodiments as shown in FIG. 3, FIG. 20 to FIG. 27, the second actuator 7000 includes a sliding rod 1, a supporting assembly 4000, a tracer 2, an axial buffering mechanism 80 and an axial limit structure 90. The second actuator 7000 is connected to the first actuator 6000 through the supporting assembly 4000, and when the first and second actuators are connected, the sliding rod 1 of the second actuator 7000 is parallel to a structure of the first actuator 6000 for connecting the cutting tool 1004. The structure of the first actuator 6000 for connecting the cutting tool 1004 includes the output shaft 400 and the joint 600, with their axes parallel to the sliding rod 1. Both the forming of the acetabular fossa/femur medullary cavity and the implantation of the prosthesis relate to a precision of an angle of the axis of the tool, and the precision of the angle of the axis is inter-related. It is more advantageous to configure the structure for connecting the cutting tool 1004 to be parallel to the structure for connecting the prosthesis 1003.

As shown in FIG. 20 to FIG. 21, the sliding rod 1 is a metal rod having a smooth surface. One end of the sliding rod 1 is configured to receive hammering by a surgeon, and the other end of the sliding rod 1 is used for connecting the prosthesis 1003. The sliding rod 1 is provided, at its middle portion, with a holding part 3. The holding part 3 is in a sleeve shape, and is sleeved on the sliding rod 1 and fixed with the sliding rod 1, to make the surgeon is capable to hold the sliding rod 1 through the holding part 3. The holding part 3 is an insulating plastic sleeve. The sliding rod 1, as a metal member, ensures a relatively high strength during transmitting of the impact. However, an over-heavy surgical instrument is not desirable, and thus the sliding rod 1 generally has a relatively small diameter, such that it is not convenient for the surgeon to hold it. The holding part 3 with the plastic material increases the diameter in the holding position of the sliding rod 1 to provide an advantageous holding condition for the surgeon, without adding a relatively large weight to the surgical tool. Certainly, in some embodiment, the holding part 3 may be an insulating rubber sleeve or a non-insulating metal sleeve. In further other embodiments, it is also possible to provide the holding part 3 without the sleeve shape, and the holding part 3 may be configured as a portion of the sliding rod 1 itself, and such portion may be larger in diameter than the diameter of a main body of the sliding rod 1 to facilitate holding.

The tracer 2 includes a tracing portion and a connecting portion. The tracing portion is provided with a plurality of positioning labels for providing position information. The positioning label may be a reflective ball or a reflective sheet which can reflect infrared light, or may be an infrared light source or an electromagnetic generator which can actively send a signal to achieve positioning. The connecting portion is used for fixing the tracer 2 to the sliding rod 1.

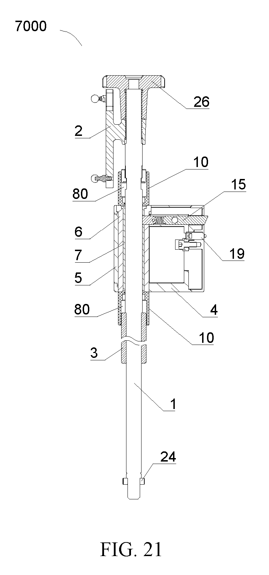

The supporting assembly 4000 includes a supporting member 4, an accommodating channel 5, an insulating sleeve 6 and a sliding sleeve 7. The supporting member 4 is generally in a hexahedron shape, with an end (such as a right end shown in FIG. 21) for connecting the robotic arm 9100. The accommodating channel 5 is a hole penetrating through the supporting member 4. The insulating sleeve 6 and the sliding sleeve 7 are each in a cylinder shape. The insulating sleeve 6 is sleeved in the accommodating channel 5 and is axially fixed to the accommodating channel 5. The insulating sleeve 6 is configured to avoid forming a conductive path between a patient and the device(s) of the robotic arm 9100 due to contact of the supporting assembly 4000 and the sliding rod 1. The sliding sleeve 7 is sleeved in the insulating sleeve 6, and is axially fixed to the insulating sleeve 6. The sliding sleeve 7 is made of metal. The sliding rod 1 and the sliding sleeve 7 form a shaft-hole fit, and there is a gap between the sliding rod 1 and the sliding sleeve 7 that allows the sliding rod 1 to slide freely with respect to the sliding sleeve 7. Thus, the sliding sleeve 7 provided between the insulating sleeve 6 and the sliding rod 1 may not only reduce abrasion of the insulating sleeve 6, but also increase smoothness of sliding of the sliding rod 1.

The axial limit structure 90 includes a check ring 9, and a first end on the holding part 3 away from the prosthesis 1003. Both the check ring 9 and the first end of the holding part 3 are fixed to the sliding rod 1, and two steps with diameters larger than a diameter of sliding rod 1 are formed on the sliding rod 1. When the sliding rod 1 moves along the sliding sleeve 7, the intervention occurs between the two steps and the supporting assembly to form axial limit to the sliding rod 1. In the present embodiment, an insulating member 10 is further provided between the check ring 9 and the supporting assembly 4000 and between the holding part 3 and the supporting assembly 4000. Therefore, the check ring 9 and the holding part 3 directly form axial intervention with the insulating member 10. The insulating member 10 is a sleeve with both ends opened. The insulating member 10 includes an inner space having a diameter larger than the diameter of the sliding rod 1. A diameter of an opening at an end of the insulating member 10 is larger than the diameter of the sliding rod 1. A diameter of an opening at the other end of the insulating member 10 is equal to the diameter of the sliding rod 1, and this end is provided with a check rim 101 to form the opening having the same diameter as that of the sliding rod 1. When the sliding rod 1 is assembled to the supporting assembly 4000, the check ring 9 and the first end of the holding part 3 are located on both sides of the supporting assembly 4000, respectively. Both the two insulating members 10 are sleeved on the sliding rod 1, and are located on both sides of the supporting assembly 4000, respectively. A side of the insulating member 10 having the check rim 101 is connected to the supporting member 4. Thus, the check ring 9 and the first end of the holding part 3 form two limit points on the sliding rod 1. When the sliding rod 1 slides with respect to the supporting assembly 4000, the check ring 9 and the first end of the holding part 3 limit a maximum sliding stroke of the sliding rod 1 with respect to the supporting assembly 4000.

In an optional embodiment, the first end of the holding part 3 in the axial limit structure 90 may be substituted by an independently arranged check ring 9. In another optional embodiment, the check ring 9 or the first end of the holding part 3 may be a step or shoulder provided on the sliding rod 1.

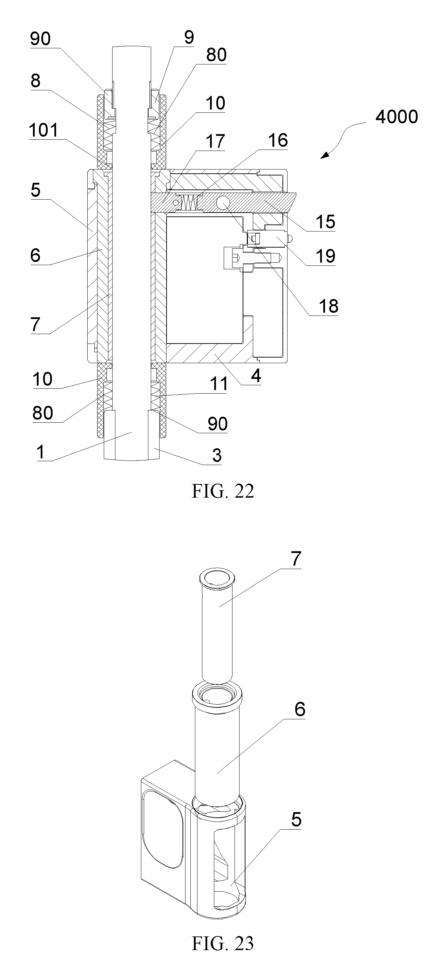

Specifically, referring to FIG. 21 and FIG. 22, an axial buffering mechanism 80 is further provided in the present disclosure, to make the sliding rod 1 and the supporting assembly 4000 form at least one buffering structure in the axial direction. In the present embodiment, the axial buffering mechanism 80 includes two buffering members, specifically being a first buffering member 8 and a second buffering member 11. The first buffering member 8 and the second buffering member 11 are arranged on both sides of the supporting assembly 4000. The two buffering members are springs. The first buffering member 8 is provided between the check ring 9 and the insulating member 10, and the second buffering member 11 is provided between the first end of the holding part 3 and the check rim 101 of the insulating member 10. Both the first buffering member 8 and the second buffering member 11 are sleeved on the sliding rod 1, and are provided in a pre-compression state in the insulating member 10. The first buffering member 8 and the second buffering member 11 produce buffering when the sliding rod 1 slides with respect to the supporting assembly 4000. When the sliding rod 1 slides, a portion of the impact on the supporting assembly 4000 is absorbed by the buffering members. Thus, when the sliding rod 1 slides axially to install the prosthesis 1003, the sliding rod 1 will not provide rigid impact on the robotic arm 9100, thereby reducing locking state or a posture error of the robotic arm 9100.

With driving by the robotic arm 9100, the second actuator 7000 achieves a target aligning posture for installing the acetabular prosthesis, and the prosthesis 1003 is aligned with the prepared acetabular fossa at the affected area of a patient. During moving and positioning of the robotic arm 9100, both the first buffering member 8 and the second buffering member 11 are in a compression state. Under the effect of the first buffering member 8 and the second buffering member t 11, the sliding rod 1 maintains a certain axial positioning relation with respect to the supporting member 4. That is, the sliding rod 1 is maintained about in a middle position of the sliding stroke, and will not move freely along the accommodating channel 5.

After the surgeon confirms that the posture of the prosthesis 1003 and the surgical path are correct, the robotic arm 9100 is configured to a mode of linear spring arm. That is, by controlling output torque of a motor at a joint of the robotic arm 9100, the robotic arm 9100 is configured such that the damping on its end arm/rod is very low in the axial direction of the sliding rod 1, but is very high in other directions. In this mode, the second actuator 7000 connected to the robotic arm 9100 may, under the action of the external force, move in the axial direction of the sliding rod 1, but it is difficult for it to move radially or rotate around an axis in the radial direction. The surgeon holds the holding part 3 in hand and applies the impact on the first end of the sliding rod 1. The impact may be applied by hitting with a hammer or a sliding weight. With the impact, the sliding rod 1 drives the prosthesis 1003 to enter the acetabulum. At a moment of the impact, the supporting assembly 4000 will not move at once because the effect of inertia. During movement of the sliding rod 1, the check ring 9 compresses the first buffering member 8, and the first buffering member 8 acts on the supporting assembly 4000, to make the supporting assembly 4000 moves axially with the sliding rod 1 in a delayed manner. The first buffering member 8 prevents a rigid contact between the spring check ring 9 and the supporting member 4. After the sliding rod 1 completes one impact on the prosthesis 1003, under the effect of the first buffering member 8, the relative relation between the sliding rod 1 and the supporting assembly 4000 automatically restores to the state before hammering. In some cases, it is further necessary to apply a force in a direction opposite to the direction of the hammering force during implanting the prosthesis to the second actuator 7000, to remove the prosthesis 1003 or a prosthesis testing sample from the acetabulum. In this case, the second buffering member 11 may prevent a rigid contact between the sliding rod 1 and the supporting assembly 4000. With configuration of the above-mentioned buffering mechanism, during impacting the sliding rod 1, the robotic arm 9100 may automatically move with the sliding rod 1, without manually holding the actuator. An operator may hold the sliding rod 1, and may sense the impact and shock, like in traditional surgeries.

A stroke of axial moving of the sliding rod 1 is defined by the first end of the holding part 3 and the check ring 9, of the limit structure. The first buffering member 8 and the second buffering member 11 are provided such that the limit structure of the sliding rod 1 is never in rigid contact with the supporting member 4. When the sliding rod 1 is not receiving the impact, the sliding rod 1 is maintained in a middle position with respect to the accommodating channel 5, and the sliding rod 1 will not freely move with respect to the supporting assembly 4000. Rather, a certain force is necessary to overcome the effect of the first buffering member 8 or the second buffering member 11 to make the sliding rod 1 move, and thus avoiding any random movement of the sliding rod 1 during movement of the robotic arm 9100.

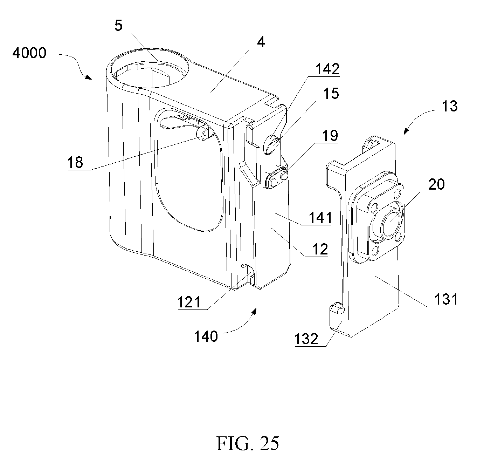

In an optional embodiment, the supporting assembly 4000 is provided thereon with a quick release mechanism 140 for connecting the second actuator 7000 with the robotic arm 9100 or the first actuator 6000. As shown in FIG. 25 to FIG. 27, the quick release mechanism 140 includes a first limit mechanism 141 and a second limit mechanism 142. The first limit mechanism 141 is an inserting block 12, and the second limit mechanism 142 is a locking pin assembly. The inserting block 12 is used for connecting with the robotic arm 9100 or the first actuator 6000 in an insert-connection manner. An insert-connection limit direction of the locking pin assembly is perpendicular to the inserting-joint direction of the inserting block 12. The inserting block 12 and the supporting member 4 are fixedly connected or integrally formed. The insert block 12 is provided, on an end thereof along its inserting direction, with two limit grooves 121 for limiting degree of freedom in the inserting-joint direction.

The supporting member 4 is provided thereon with an installing hole 14 for accommodating the locking pin assembly, and the installing hole 14 communicates with the accommodating channel 5. The locking pin assembly includes a locking pin 15, a first elastic member 16, a spacer block 17 and a locking pin pulling bolt 18. The spacer block 17, the first elastic member 16 and the locking pin 15 are provided in sequence in the installing hole 14. The first elastic member 16 is a spring. The spacer block 17 abuts against the sliding rod 1. The locking pin 15 is in the installing hole 14 and penetrates through the inserting block 12 perpendicularly in a thickness direction of the inserting block 12. The first elastic member 16 is in a compressed state, and is provided between the locking pin 15 and the spacer block 17. A middle section of the installing hole 14 communicates with an outside of the supporting member 4, and forming a movable region in which the locking pin 15 is capable to be manually operated. The locking pin pulling bolt 18 radially penetrates through the locking pin 15, and is fixed with the locking pin 15. The locking pin 15 is restricted in the movable region by the locking pin pulling bolt 18. With pushing by the first elastic member 16, the locking pin pulling bolt 18 abuts against an end of the movable region, and a head of the locking pin 15 penetrates through a surface of the inserting block 12. The head of the locking pin 15 is an inclined face.

In order to install the second actuator 7000 to the first actuator 6000 by the quick release mechanism 140, the first actuator 6000 is provided thereon with a second connector 13 in an inserting slot form. Specifically, the second connector 13 includes a bottom plate 131, a locking pin hole 133 and a limit button 132, where the bottom plate 131 is in a rectangular shape. The locking pin hole 133 is provided in a thickness direction of the bottom plate 131. The number of the limit buttons 132 is four, and they are provided at four corners of the bottom plate 131, respectively. The limit buttons 132 and the bottom plate 131 form the second connector 13. The limit button 132 specifically includes a first section 1321 and a second section 1322 which are connected to each other. The first section 1321 is connected to the bottom plate 131, and is perpendicular to the bottom plate 131. The second section 1322 is parallel to the bottom plate 131, and extends towards an inside of the bottom plate 131. The limit buttons 132 and the bottom plate 131 form a space accommodating the inserting block 12. Moreover, when the inserting block 12 is inserted and connected into the second connector 13, the limit groove 121 is in clamping-connection with the limit button 132, and with the limitation by the limit buttons 132, the inserting block 12 cannot be disengaged from the clamping groove in the insert-connection direction.

By configuration of the quick release mechanism 140, it may be convenient to assemble/disassemble the second actuator 7000. As shown in FIG. 25 to FIG. 27, when the inserting block 12 is connected to the second connector 13 from top to bottom, the plane of the bottom plate 131 is first fitted the plane of the inserting block 12, the inclined face of the head of the locking pin contacts with the bottom plate 131, and the locking pin 15 retracts towards the supporting member 4. The supporting member 4 is moved downwards with respect to the second connector, the limit groove 121 and limit button 132 are in clamping-fitted, the head of the locking pin 15 enters the locking pin hole 133, and the inserting block 12 and the second connector 13 are completely fitted. In the spatial rectangular coordinate system, the fitness in thickness and width between the inserting block 12 and the second connector 13 defines five degrees of freedom of the inserting block 12, except a z axis (or maybe an x axis or a y axis), the clamping-fitting between the limit groove 121 and the limit button 132 defines a degree of freedom of the second actuator 7000 sliding along the z axis in a first direction, and the fitting between the locking pin 15 and the locking pin hole 133 enables a degree of freedom of the second actuator 7000 sliding along the z axis in a second direction. In FIG. 25 to FIG. 27, the first direction is a downward direction along the axial direction of the accommodating channel 5, and the second direction is an upward direction along the accommodating channel 5. By now, the second actuator 7000 is fixedly connected to the first actuator 6000 by the configuration of the inserting block 12, the second connector 13 and the locking pin assembly. For disassembling, it is only necessary to move the locking pin pulling bolt 18 (moving to the left in FIG. 25) to remove the head of the locking pin 15 from the locking pin hole 133, and then pull out (pulling upwards with respect to the second connector 13 in FIG. 25) the inserting block 12 from the second connector 13. With the configuration of the quick release mechanism 140 for the second actuator 7000, a surgeon during surgery may complete installing and removing of the second actuator 7000 quickly, and thus saving surgery time.

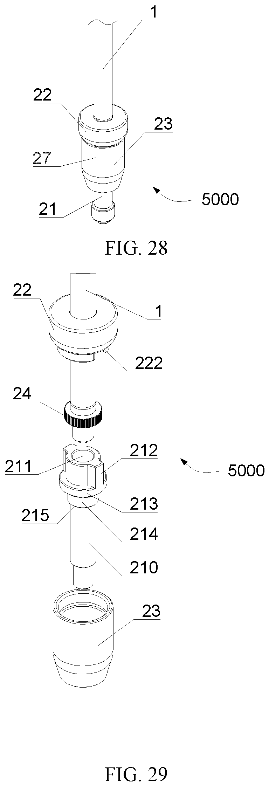

As shown in FIG. 28, in an optional embodiment, the second actuator 7000 further includes an adjusting assembly 5000. The adjusting assembly 5000 connects the prosthesis 1003 to the sliding rod 1, and is capable to adjust a circumferential position of the prosthesis 1003 with respect to the sliding rod 1. The adjusting assembly 5000 includes an adapter shaft 21 and an adjusting member 27. An end of the adapter shaft 21 is connected to the sliding rod 1, and the other end of the adapter shaft 21 is connected to the prosthesis 1003 of the hip joint. The adjusting member 27 is sleeved on a joint between the adapter shaft 21 and the sliding rod 1. With the effect of an external force, the adjusting member 27 may move between a first position 28 and a second position 29 of the adapter shaft 21. The adjusting member 27 is circumferentially fixed with the sliding rod 1 at the first position 28, and the adjusting member 27 is circumferentially adjustable with respect to the sliding rod 1 at the second position 29.

As shown in FIG. 29, the adapter shaft 21 includes a sliding rod connection joint, a main shaft section 210 and an acetabulum prosthesis connection joint. The sliding rod connection joint and the acetabulum prosthesis connection joint are provided at both ends of the main shaft section 210, respectively. The sliding rod connection joint is used for connecting with the sliding rod 1. The acetabulum prosthesis connection joint is used for connecting the prosthesis 1003.

The sliding rod connection joint is provided, at its top end, with a connecting hole 211. The connecting hole 211 is a smooth hole, and it is provided, on its outer periphery, with two clamping blocks 212 which are symmetrical with respect to the axis of the adapter shaft 21 and radially extends in a form of a Chinese character "-". Below the clamping block 212, a flange 213 is provided, having a radius equal to the maximum radius of the clamping block 212. Below the flange 213, a limit section 214 is provided, having a radius larger than a radius of the main shaft section 210. Moreover, a limit step 215 is formed at a joint between the limit section 214 and the main shaft section 210.

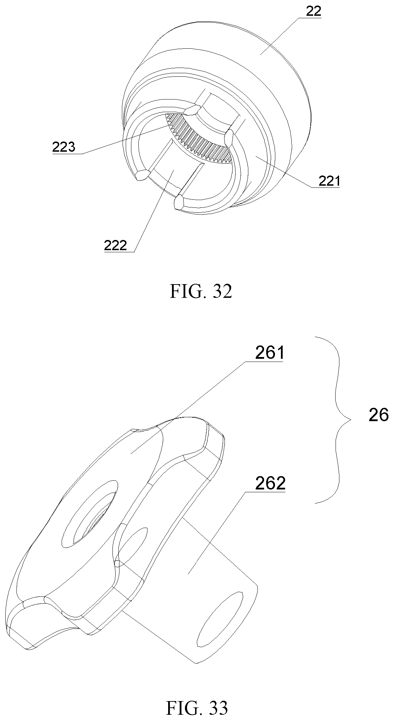

Referring to FIG. 29 to FIG. 32, the adjusting member 27 includes a nut 22 and a reducing sleeve 23 detachably connected, a spline 24 and a holding member 25. Specifically, referring to FIG. 32, the nut 22 is in a shell shape with a downward opening, and includes an outer wall 221 which is provided thereon, at the opening, with an outer thread, and is also provided thereon with two clamping grooves 222 symmetrically. The clamping groove 222 extends into the nut 22. A spline keyway 223 is provided in a position in the nut 22 close to the bottom. The reducing sleeve 23 is in a cup shape with an opening, and is provided, on its inner wall at the opening, with an inner thread. The spline 24 is fixed on the sliding rod 1, and is provided on its outer periphery with a toothed bulge. The holding member 25 is a resilient spring.

In the connected state, the nut 22 is sleeved on the sliding rod 1 above the spline 24, the reducing sleeve 23 is sleeved on the adapter shaft 21, and the reducing sleeve 23 and the nut 22 are connected by fitting of the inner thread and the outer thread. The holding member 25 is provided in the reducing sleeve 23, with one end abutting against the bottom of the reducing sleeve 23 and the other end abutting against the flange 213.

In use, the end of the sliding rod 1 is inserted into the connecting hole 211, and the nut 22 and the reducing sleeve 23 form an integrated structure by thread connection. To facilitate understanding, the explanation will be provided hereinafter in combination with the operation state and the adjusting process of the adjusting member 27.

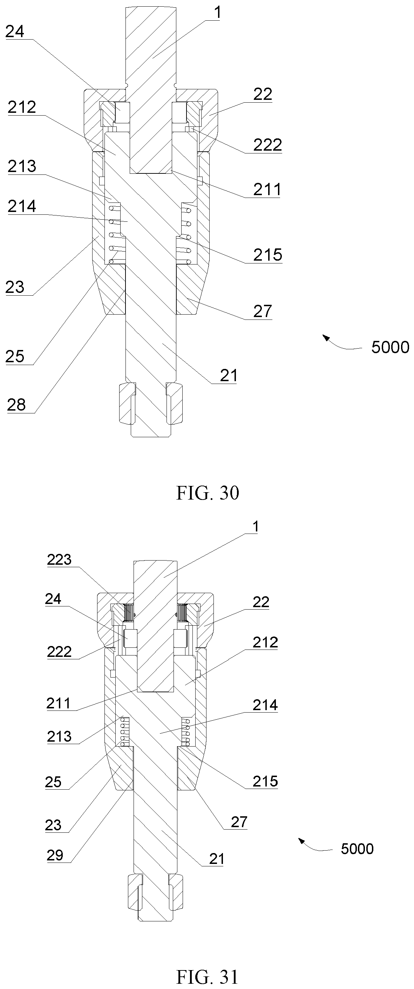

In the operation state, the adjusting member 27 is in the first position 28. As shown in FIG. 30, the holding member 25 is in the compressed state and abuts against with the flange 213 and the bottom of the reducing sleeve 23. The holding member 25 pulls the nut 22 through the reducing sleeve 23, to make the spline keyway 223 of the nut 22 is connected to the spline 24. The clamping block 212 is embedded in the clamping groove 222. Thus, the sliding rod 1 and the adjusting device are circumferentially fixed by connection of the spline 24 and the spline keyway 223, and the adapter shaft 21 and the adjusting apparatus are circumferentially fixed by cooperation of the clamping block and the clamping groove 222. Based on the above-mentioned process and principle, in the operation state, by the connection of the adjusting assembly 4000, the sliding rod 1 and the adapter shaft 21 are fixed axially, radially and circumferentially.

In order to meet clinic requirements, when the prosthesis 1003 is implanted into the prepared acetabular fossa of an affected area of a patient, it is necessary to ensure that the prosthesis 1003 is in the correct installing direction. For example, for the prosthesis 1003 having a wing part, it is necessary for it to be fixed to the acetabular fossa to strengthen the structure at the acetabular fossa, and it is also necessary for the wing part to be connected to the acetabular fossa in the correct direction. Therefore, each time before the sliding rod 1, it is necessary to adjust the direction of the prosthesis 1003. Based on the second actuator 7000 in the present embodiment, when the direction of the prosthesis 1003 is adjusted, as shown in FIG. 31, a surgeon pulls the adjusting device upwards to make it overcome the elastic force of the holding member 25 until the bottom of the reducing sleeve 23 abuts against the limit step 215. The adjusting member 27 is located in the second position 29. At this time, the spline 24 is disengaged from the spline keyway 223, and the clamping block 212 is not removed from the clamping groove 222, the adjusting member 27 may circumferentially rotate with respect to the sliding rod 1, and the adapter shaft 21 rotates with rotation of the adjusting member 27. Thus, it is possible to achieve the adjustment the direction of the prosthesis 1003 with respect to the sliding rod 1 only by rotation of the adjusting member 27, without rotation of the sliding rod 1. Further, as the sliding rod 1 is connected to a tracer 2 for providing position information of the sliding rod 1 in real time, and it is necessary for the tracer 2 to be aligned with the positioner for receiving the position information. Therefore, the above-mentioned configuration of the adjusting assembly 5000 also ensures that when the prosthesis 1003 is adjusted, the tracer 2 fixedly connected to the sliding rod 1 will not fail in aligning with the positioner due to rotation of the sliding rod 1, and thus ensuring that the tracer 2 is capable to be identified by the positioner in real time.

Furthermore, based on the adjusting assembly 5000, by changing the acetabulum prosthesis connection joint of the adapter shaft 21, the adapter shaft 21 may connect with the prostheses 1003 of various models from different manufacturers. Therefore, it is not necessary to replace the whole sliding rod 1 to adapt to a different prosthesis 1003, and thus the adaptation and application range of the second actuator 7000 is improved.

In an optional embodiment, the buffering member may include only the first buffering member 8, and omitting the second buffering member 11.

In some optional embodiments, it is possible to provide one buffering member, such as the buffering member 8. Two ends of the buffering member 8 are connected to the check ring 9 and the supporting assembly 4000, respectively. Therefore, the sliding rod 1 will be pulled or stopped by the buffering member 8 when it moves in either of the two directions, and thus forming buffering and possibly driving the supporting assembly 4000 to move with the sliding rod 1.

In some optional embodiments, the two buffering members of the axial buffering mechanism 80 may not be pre-compressed. For example, the first buffering member 8 may be compressed only by effect of gravity of the sliding rod 1. Lengths of the two buffering members may be less than the stroke of the sliding rod 1, and the buffering members may move between the limit structures as long as any rigid collision is capable to be avoided.

In some optional embodiments, refer to FIG. 20 and FIG. 33. A cap nut 26 is provided at an end of the sliding rod 1, for receiving an impact force. The cap nut 26 includes a force-bearing plate 261 and a connecting section 262, and the connecting section 262 is fixedly connected to the sliding rod 1 by thread. Certainly, the connection manner is not limited to the threaded connection, it may be other connection manners such as pin connection. An area of the force-bearing plate 261 is larger than an area of the end of the sliding rod 1, so that the force-bearing plate 261 provides a larger force-bearing target for hitting when an impact force is applied by a surgeon, to avoid a phenomenon of missing hitting due to the relatively small area at the end of the sliding rod 1.

With continued reference to FIG. 1, in a second aspect of the present disclosure, a surgical system is provided, including: the actuator for the hip replacement 9300 as provided in the first aspect, a robotic arm 9100, a navigation system 9000 and a controlling system 9200. The robotic arm 9100 is configured to carry the actuator for the hip replacement, the navigation system 9000 is used for measuring a position of the actuator for the hip replacement 9300, and the controlling system 9200 is configured to drive the robotic arm 9100 to move the actuator for the hip replacement 9300 to a target position according to a surgical plan.

The robotic arm 9100 may completely actively control the orientation of the actuator, and may also limit some degree(s) of freedom of movement or range of movement. of the actuator, in a cooperation manner. Specifically, by programming of the controlling system 9200, it is possible to control the robotic arm 9100 to make it completely independently move according to the surgical plan, or provide tactile feedback or force feedback to prevent the surgeon from manually moving beyond a predetermined virtual boundary, or provide virtual guidance to guide the surgeon to move in a certain degree of freedom. The virtual boundary and the virtual guidance may come from the surgical plan, or may be configured by an input device during surgery. The actuator is detachably connected to the robotic arm 9100.

The navigation system 9000 is used for measuring the positions of the actuator and a patient. The navigation system 9000 generally includes a positioner and a tracer. The tracers are installed on the actuator, a surgical tool and a body of a patient. Generally, the tracer is an array consisting of a plurality of tracing elements, and each tracing element may transmit an optical or electromagnetic signal or the like in an active or passive manner. The positioner (such as a binocular camera) measures the orientation of the above-mentioned tracer by 3D measuring technique.

The controlling system 9200, according to the position information obtained by the navigation system 9000, compares the current position and a target position of the actuator, and drives the robotic arm 9100 to move the actuator for the hip replacement 9300 to the target position according to the surgical plan. In the surgical plan, it is possible to include a moving path, a movement boundary, and the like of the robotic arm. The surgical plan is recorded in a three-dimensional (3D) reconstruction digital model for an affected bone of a patient, and the digital model will be registered with the tissue of the patient during surgery.

In the surgical system, with the assistance of the robotic arm 9100, the controlling system 9200 and the navigation system 9000, it is capable to perform preparation of the acetabular fossa or the medullary cavity at an affected area, with only the first actuator 6000 connected, and it is capable to perform installing of the prosthesis 1003, with the second actuator 7000 connected to the first actuator 6000.

FIG. 34 to FIG. 42 show structural schematic diagrams of the actuator for installing the prosthesis in another embodiment. In the present embodiment, the actuator for installing the prosthesis differs from the actuator as shown in FIG. 20 to FIG.22 mainly in that a first damping mechanism is added between the sliding rod 1 and the accommodating channel 5, and the first damping mechanism is capable to at least provide radial buffering. The actuator for installing the prosthesis may be used in combination with the above-described prosthesis forming actuator.

It is found in research by an inventor that during a hip replacement surgery with robot assistance, the surgery device includes: an acetabulum prosthesis (acetabular cup), an actuator for installing a prosthesis, a bone hammer and a robotic arm or the like. The actuator for installing the prosthesis includes a connecting mechanism for connecting the robotic arm and a sliding rod connected to the connecting mechanism. During surgery, it is necessary to use the hammering on the sliding rod 1 in the actuator for installing the prosthesis by the bone hammer to hammer the acetabulum prosthesis into the acetabular fossa of a patient. The shock (also called impact) during hammering is transmitted from the sliding rod 1 to the connecting mechanism, and then further to the robotic arm 9100. When the hammering force is high and the shock is violent, there will be a risk of locking fault for the robotic arm. The locking fault of the robotic arm will not only damage the robotic arm itself, but also decrease reliability of the robotic arm, and further will affect smooth of a surgery process and put the patient in danger. Based on the study on the above-mentioned problem, an actuator for installing a prosthesis is provided by the inventor, which may effectively solve the problem that the impact received by the actuator for installing the prosthesis is easily transmitted to the robotic arm, and thus the reliability of the robotic arm connected to the actuator for installing the prosthesis is improved.

In order to better understand the present disclosure, an actuator for installing a prosthesis according to the embodiment of the present disclosure will be described in details hereinafter in combination with FIG. 34 to FIG. 42.

Please refer to FIG. 34 to FIG. 36. In the embodiment of the present disclosure, an actuator for installing a prosthesis 1 is provided, configured to perform a surgical plan with holding by the robotic arm 9100. This prosthesis installing actuator 1 includes a sliding rod 11, a supporting assembly 10 and a first damping mechanism 12. The sliding rod 11 is used for carrying a prosthesis and is capable to receive the impact to install the prosthesis to a target position. The supporting assembly 10 includes an accommodating channel 101. The accommodating channel 101 is used for accommodating the sliding rod 11, and is configured such that the sliding rod 11 is capable to move linearly with respect to the accommodating channel 101. A diameter of the accommodating channel 101 is larger than a diameter of the sliding rod 11. The first damping mechanism 12 is provided in the accommodating channel 101, and the first damping mechanism 101 is configured to join the sliding rod 11 on a circumferential side, for reducing the impact transmitted from the sliding rod 11 to the supporting assembly 10. The first damping mechanism 12 is capable to resiliently hold the sliding rod 11 in the accommodating channel 101, and is capable to make the sliding rod 11 and the accommodating channel 101 maintain substantially coaxial and do not contact with each other. When the sliding rod 101 is deflected or moved radially by a radial force, it will not directly collide rigidly with the supporting assembly 10 due to elastic deformation of the first damping mechanism 12, and thus a buffering requirement and a requirement of positioning precision of the system are both met.

Where, the prosthesis is the acetabulum prosthesis, also called an acetabular cup. The supporting assembly 10 is configured to connect with the robotic arm and the robotic arm holds the sliding rod 11 by connecting with the supporting assembly 10. In the actuator for installing the prosthesis as provided in the present disclosure, the first damping mechanism 12 may achieve installation of the first damping mechanism 12 and the supporting assembly 10 by interference fitting with the accommodating channel 101. The supporting assembly 10 may be slidably connected to the sliding rod 11, and defines a sliding stroke of the sliding rod 11 in a length direction x of the sliding rod 11 by the limit mechanism.

In the actuator for installing the prosthesis as provided in the present disclosure, the accommodating channel 101 in the supporting assembly 10 is used for accommodating the sliding rod 11 and the first damping mechanism 12. The sliding rod 11 penetrates through the accommodating channel 101, that is, the supporting assembly 10 is sleeved on the sliding rod 11. The first damping mechanism 12 is provided between the sliding rod 11 and the accommodating channel 101. The first damping mechanism 12 is provided on a circumferential side of the sliding rod 11, it is possible to use the circumferential side of the sliding rod 11 to sufficiently reduce the impact transmitted from the sliding rod 11 to the supporting assembly 10, therefore the impact influence on the robotic arm connected to the supporting assembly 10 is effectively reduced, and thus probability of locking fault of the robotic arm connected with the actuator for installing the prosthesis 1 due to the impact is decreased, and consequently, the reliability of the robotic arm is improved, and also probability of success of the surgery is increased.

In a possible embodiment, as shown in FIG. 34, the actuator for installing the prosthesis 1 further includes a hitting cap 13 and an acetabular cup connecting mechanism 14. The hitting cap 13 is provided on an end of the sliding rod 11, and the acetabular cup connecting mechanism 14 is provided at the other end of the sliding rod 11. The acetabular cup connecting mechanism 14 is used for connecting the prosthesis.

In the actuator for installing the prosthesis 1 as provided in the present disclosure, the hitting cap 13 is connected to one end of the sliding rod 11, and the prosthesis connecting mechanism 14 is connected to the other end of the sliding rod 11. During use of the actuator for installing the prosthesis 1, that is, during surgery, the hitting cap 13 is the portion receiving the force of hammering. The sliding rod 11 and the hitting cap 13 are fixed to form an integrated structure. By hammering the hitting cap 13 by a bone hammer, the prosthesis connected to the acetabular cup connecting mechanism 14 is installed into an acetabular fossa of a patient.

In a possible embodiment, the first damping mechanism 12 includes a first elastic member 122. The first elastic member 122 has ability of deformation in the radial direction and/or axial direction of the accommodating channel 101, and thus is capable to absorb the deformation in the radial direction and/or axial direction. When the sliding rod 11 receives the impact, the first elastic member 122 may radially and/or axially reduce transmission of the impact on the sliding rod 11 to the supporting assembly 10, and thus protecting the robotic arm connected to the supporting assembly 10.

In a possible embodiment, the first damping mechanism 12 further includes a sleeve 121, with its inner circumference in shaft-hole fit with the sliding rod 11 and its outer circumference fitting with the first elastic member 122.

In the above-mentioned embodiment, the sleeve 121 is sleeved on the circumferential side of the sliding rod 11, and the first elastic member 122 is provided on a circumferential side of the sleeve 121, to achieve buffering between the sliding rod 11 and the supporting assembly 10 comprehensively.

In a possible embodiment, the first elastic member 122 is a damping pad which is in a ring shape and has a predetermined axial thickness and radial thickness.

In the above-mentioned embodiment, the first elastic member 122 is the damping pad, and its material may be silica gel. The material of silica gel has a relatively good elasticity, and may provide good buffering and damping effect, with such material widely available and convenient for installing. The damping pad may deform in response to impact to achieve shock absorption, thus reducing transmission of the shock from the sliding rod 11 to the supporting assembly 10. The damping pad may in a ring shape such that it is capable to be sleeved on the circumferential side of the sleeve 121. The damping pad may be installed to the sleeve 121 in an interference fitting manner, to improve connecting compactness and better absorb the impact transmitted from the sliding rod 11 to the sleeve 121. The damping pad has a predetermined axial thickness and radial thickness, and thus may provide buffering in the axial and/or radial direction. It is possible to configure the axial thickness and the radial thickness of the damping pad according to the impact in a practical operation, and they are not specifically defined in the present disclosure.

In a possible embodiment, an outer periphery of the damping pad and the accommodating channel 101 are in a shaft-hole fit, and an inner periphery of the damping pad and the sleeve 121 are in the shaft-hole fit.

Since the damping pad is in a ring shape, the installation of the damping pad may be realized through the shaft-hole fit. Specifically, the damping pad may be installed to the sleeve 121 in an interference fitting manner, and the supporting assembly 10 may be installed to the damping pad in an interference fitting manner, so that tightness connection therebetween the three may be realized. At this point, since the tightness fit of the damping pad, the sleeve 121 and the supporting assembly 10, so that the sealing degree among the three may be improved, and thus the amount of liquid that enters the actuator for installing the prosthesis 1 may be reduced during cleaning and steam sterilization process.

In the above-mentioned embodiment, the damping pad is sleeved on the circumferential side of the sleeve 121, and the sleeve 121 is sleeved on the circumferential side of the sliding rod 11. Thus, the shock absorption function in the circumferential direction of the sliding rod 11 may be achieved. Moreover, as the damping pad achieves the shock absorption function by elastic deformation under pressure and it may deform under pressure in any direction, the shock absorption effect is capable be available in any direction except for the circumferential direction. That is, the first damping mechanism 12 may reduce the shock transmitted from the sliding rod 11 to the supporting assembly 10 comprehensively in multiple directions.

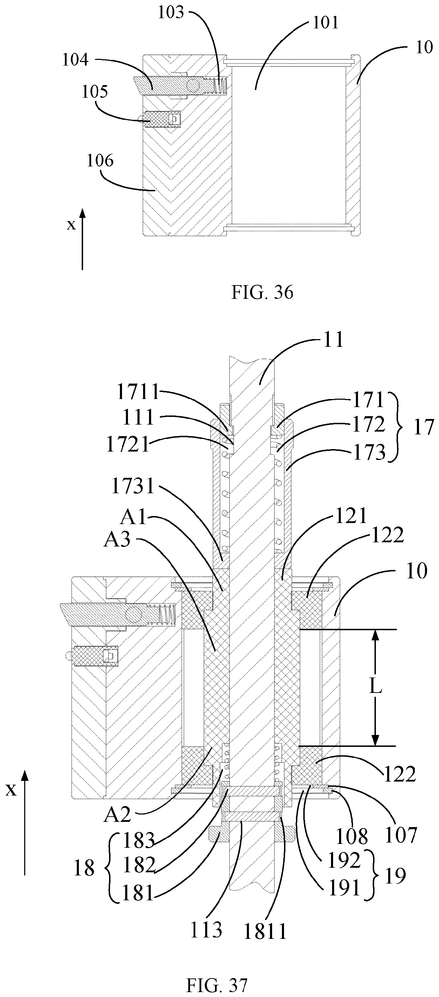

In a possible embodiment, as shown in FIG. 37, the sleeve 121 includes a first end A1 and a second end A2 in the length direction x of the sliding rod 11. There are two damping pads, where one damping pad is sleeved at the first end A1 and the other damping pad is sleeved at the second end A2. In the length direction x of the sliding rod 11, a preset gap L is formed between the two damping pads.

In the above-mentioned embodiment, there are two damping pads which are arranged in the length direction x of the sliding rod 11. In the length direction x of the sliding rod 11, the preset gap L is formed between the two damping pads, such that the two damping pads are independent with respect to each other. When the shock is produced, the two damping pads may deform individually to achieve shock absorption, without interference with each other, such that each damping pad may deform more flexibly. The two damping pads may effectively reduce transmission of the shock, respectively, and thus the shock absorption effect of the first damping mechanism 12 is improved.

In the above-mentioned embodiment, the damping pad may be installed to the sleeve 121 in an interference fitting manner, and the damping pad may be installed to the supporting assembly 10 in the interference fitting manner. In this case, as the damping pad, the sleeve 121 and the supporting assembly 10 are tightly fitted, so that tightness therebetween may be improved, and thus the possibility of infiltration of liquid from the preset gap L is decreased and reliability is improved.

In another possible embodiment, the number of the damping pad may be one. The damping pad extends from the first end A1 to the second end A2 of the sleeve 121. Compared with the configuration of two damping pads, the amount of the used material is increased, but the number of the parts is decreased, so that complexity of assembling is lowered, and the shock absorption effect is remained, reducing the impact transmitted from the sliding rod 11 to the supporting assembly 10.

In an optional embodiment, the first elastic member 122 may be a group of springs arranged in a circumferential direction of the sliding rod 11, and a direction of deformation of the springs is a radial direction of the accommodating channel. The springs are mainly configured to provide radial shock absorption between the sliding rod 11 and the supporting assembly 10, and thus protecting the robotic arm connected to the supporting assembly 10.

In a possible embodiment, as shown in FIG. 37, the sleeve 121 includes a middle region A3 located between the first end A1 and the second end A2. Diameters of the first end A1 and the second end A2 are less than a diameter of the middle region A3 to form grooves, and the damping pad partially extends into the groove.

In the above-mentioned embodiment, the diameters of the first end A1 and the second end A2 of the sleeve 121 refer to diameters of circumcircles of the first end A1 and the second end A2 of the sleeve 121 along their own circumferential sides, and the diameter of the middle region A3 refers to a diameter of a circumcircle of the middle region A3 along its own circumferential side.

In the above-mentioned embodiment, by configuring the diameters of the first end A1 and the second end A2 of the sleeve 121 as being less than the diameter of the middle region A3, the grooves may thus be formed at the first end A1 and the second end A2, respectively. That is, stepped surfaces are formed between the first end A1 and the middle regionA3 and between the second endA2 and the middle regionA3. This stepped surface is an inner surface of the groove. By configuring a cross-section in the damping pad, parallel to the length direction x of the sliding rod 11, as an "L" shape, such that one end of the L shape contacts with the stepped surface and the other end of the L shape contacts with an outer surface of the middle region A3, limit to the damping pad in the length direction x of the sliding rod 11 is achieved, and thus ensuring the two damping pads achieve the shock absorption effect at two ends of the sleeve 121.

In a practicable embodiment, the first damping mechanism 12 also includes an axial limit structure 19, which is configured to prevent the first damping mechanism 12 from axially moving out of the accommodating channel 101.

In the above-mentioned embodiment, the number of the axial limit structure 19 may be two, and the two axial limit structures 19 are respectively provided at two ends of the sleeve 121 to limit the first damping mechanism 12 in the accommodating channel 101.

When the first damping mechanism 12 is located at the "L" shape damping pad above-mentioned, the axial limit structures 19 may be provided on a side of the first damping mechanism 12 away from the middle regionA3, and is connected to the sleeve 121 and the supporting assembly 10, separately. The axial limit structures 19 are provided on the side of the first damping mechanism 12 away from the middle region A3, so that the axial limit structures 19 may prevent the damping pad from moving out of the accommodating channel 101. The position of the damping pad along the length direction x of the sliding rod 11 is thoroughly fixed through the limiting of the axial limit structures 19 and the stepped surfaces, so that the damping pad may be prevented from moving along the length direction x of the sliding rod 11. In a practicable embodiment, as shown in FIG. 37, the axial limit structure 19 includes a snap spring 191 and a snap spring pad 192, the snap spring pad 192 contacts with the damping pad, the snap spring 191 is located on a side of the snap spring pad 192 away from the damping pad, and the snap spring 191 is in clamping-connection with the sleeve 121 and the supporting assembly 10.

In the above-mentioned embodiment, the supporting assembly 10 may be provided with snap spring pad grooves 107 for accommodating the snap spring pads 192, and may be provided with snap spring grooves 108 for accommodating the snap springs 191. Both the snap spring pad grooves 107 and the snap spring grooves 108 extend along the circumferential direction of the accommodating channel 101. The snap spring grooves 108 are located between two end surfaces of the supporting assembly 10 opposite to each other along the length direction x of the sliding rod 11, so that the snap springs 191 may be effectively limited along the length direction x of the sliding rod 11.

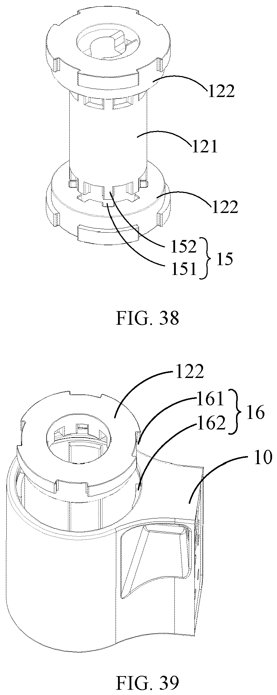

In a practicable embodiment, as shown in FIG. 38, a first circumferential direction limit structure 15 is formed between the damping pad and the sleeve 121, and the first circumferential direction limit structure 15 includes first grooves 151 and first protrusions 152 that fit with each other.

There are a plurality of the first grooves 151, and the plurality of the first grooves 151 are formed in an inner side of the damping pad and arranged in a circumferential direction of the damping pad. The number of the first protrusions 152 is the same as the number of the first grooves 151, and the first protrusions 152 are formed on an outer side of the sleeve 121 and arranged in the circumferential direction of the sleeve 121.

In the above-mentioned embodiment, the first circumferential direction limit structure 15 is used for limiting the position between the damping pad and the sleeve 121 in the circumferential direction of the sleeve 121, thereby reducing or even avoiding the relative position change of the sleeve 121 and the damping pad in the circumferential direction of the sleeve 121. The first circumferential direction limit structure 15 includes the first grooves 151 and the first protrusions 152 that fit with each other. The first protrusions 152 may be formed on the outer side of the sleeve 121, for example, the side of the sleeve 121 faces away from the accommodating channel 101, and in this case, the first grooves 151 are formed on the inner side of the damping pad, that is, the side of the damping pad faces towards the sleeve 121. The first grooves 151 and the first protrusions 152 are arranged in a one-to-one correspondence manner, and there are a plurality of the first grooves 151, and the plurality of first grooves 151 are arranged in the circumferential direction of the damping pad. There are a plurality of the first protrusions 152, and the plurality of first protrusions 152 are arranged in the circumferential direction of the sleeve 121. Alternatively, the first grooves 151 may be formed on the outer side of the sleeve 121, for example, the side of the sleeve 121 faces away from the accommodating channel 101, and in this case, and the first protrusions 152 are formed on the inner side of the damping pad, that is, the side of the damping pad facing the sleeve 121.

In the above-mentioned embodiment, the first protrusions 152 are formed at a first end A1 and a second endA2 of the sleeve 121 and extends to an end face of the sleeve 121 in a length direction x of the sleeve 121. The damping pad may be slid into the first end A1 or the second end A2 along the length direction x of the sleeve 121 to fit with the first protrusions 152 and ensure sealing reliability, so that the installing manner is simple.

In the above-mentioned embodiment, by providing the first circumferential direction limit structure 15, the probability of relative position change between the damping pad and the sleeve 121 along the circumferential direction of the sleeve 121 under an action of an external force may be reduced, so that influence of the sealing performance and the structural stability of the first damping mechanism 12 due to the relative position change between the damping pad and the sleeve 121 along the circumferential direction of the sleeve 121 is reduced.

In a practicable embodiment, as shown in FIG. 39, a second circumferential direction limit structure 16 is formed between the damping pad and a housing 101, and the second circumferential direction limit structure 16 includes second grooves 161 and second protrusions 162 that fit with each other.

There are a plurality of the second groove 161, and the plurality of second grooves 161 are formed on the outer side of the damping pad and arranged along the circumferential direction of the damping pad. The number of the second protrusions 162 is the same as the number of the second grooves 161, and the second protrusions 162 are formed on a side of the housing 101 facing towards the accommodating channel 101 and arranged in the circumferential direction of the accommodating channel 101.

In the above-mentioned embodiment, the second circumferential direction limit structure 16 is used for limiting the position between the damping pad and the housing 101 in the circumferential direction of the sleeve 121, thereby the relative position change of the housing 101 and the damping pad in the circumferential direction of the sleeve 121 is reduced. The second circumferential direction limit structure 16 includes the second grooves 161 and the second protrusions 162 that fit with each other. The second protrusions 162 may be formed on the outer side of the damping pad, for example, the side of the damping pad faces away from the sleeve 121, and in this case, the second grooves 161 are formed on the inner side of the housing 101, that is, the side of the housing 101 faces towards the accommodating channel 101. The second grooves 161 and the second protrusions 162 are arranged in a one-to-one correspondence manner. There are a plurality of the second grooves 161, and the plurality of second grooves 161 are arranged in the circumferential direction of the accommodating channel 101. There are a plurality of the second protrusions 162, and the plurality of second protrusions 162 are arranged in the circumferential direction of the damping pad. Alternatively, the second grooves 161 may be formed on the outer side of the damping pad, for example, the side of the damping pad facing away from the sleeve 121, and in this case, the second protrusions 162 are formed on the inner side of the housing 101, that is, the side of the housing 101 facing the accommodating channel 101.

In the above-mentioned embodiment, the housing 101 may be assembled with the damping pad along the axial direction of the accommodating channel 101, so that the sealing reliability between the sealing housing 101 and the damping pad may be ensured, and the installing manner is simple.

In the above-mentioned embodiment, by providing the second circumferential direction limit structure 16, the probability of relative position change between the damping pad and the housing 101 along the circumferential direction of the accommodating channel 101 under an action of an external force may be reduced, so that the influence of the sealing performance and the structural stability of the first damping mechanism 12 due to the relative position change between the damping pad and the housing 101 along the circumferential direction of the accommodating channel 101 is reduced.

In a practicable embodiment, as shown in FIG. 37, the actuator for installing the prosthesis 1 further includes a second damping mechanism 17 and a third damping mechanism 18 respectively provided on two sides of the first damping mechanism 12 along the length direction x of the sliding rod 11.

The second damping mechanism 17 and the third damping mechanism 18 are separately connected to the sliding rod 11, and limit the first damping mechanism 12 in the length direction x of the sliding rod 11.

The supporting assembly 10 may be slidably connected to the sliding rod 11, and limits a relative position of the connecting mechanism 10 along the length direction X of the sliding rod 11 through the second damping mechanism 17 and the third damping mechanism 18. When the sliding rod 11 is hit, vibration in the length direction x of the sliding rod 11 may be reduced through the second damping mechanism 17 and the third damping mechanism 18, so that the damping performance between the sliding rod 11 and the supporting assembly 10 in the actuator for installing the prosthesis 1 is further improved, and thus the reliability of the robotic arm connected to the supporting assembly 10 is improved.

The impact of the second damping mechanism 17 and the third damping mechanism 18 on the supporting assembly 10 in the length direction x of the sliding rod 11 may also be absorbed by the damping pad in the first damping mechanism 12, thereby the damping performance of the actuator for installing the prosthesis 1 is further improved.

In a practicable embodiment, each of the second damping mechanism 17 and the third damping mechanism 18 includes a second elastic member. The second elastic member has a capability of deforming in the axial direction of the sliding rod 11, and is configured to reduce axial impact transmitted from the sliding rod to the supporting assembly 10.

The second elastic member may include a pressure spring, specifically, the second elastic member in the second damping mechanism 17 may include a first pressure spring 171, and the second elastic member in the third damping mechanism 18 may include a second pressure spring 181.

In a practicable embodiment, as shown in FIG. 37, the second damping mechanism 17 includes a first limit block 171, a first pressure spring 172 and a pressure spring cover 173. The sliding rod 11 includes a third groove 111 extending in the circumferential direction. The first limit block 171 includes a second clamping end 1711, and the second clamping end 1711 is configured to be clamping-fitted with the third groove 111. The first pressure spring 172 is sleeved on the circumferential side of the sliding rod 11 and includes a first clamping end 1721 in contact with the first limit block 171, and the other end of the first pressure spring 172 abuts against the first damping mechanism 12 through a bottom of the pressure spring cover 173. The pressure spring cover 173 is sleeved on the circumferential side of the first pressure spring 172, and includes a third clamping end 1731 extending between the first pressure spring 172 and the first damping mechanism 12.

The sliding rod 11 is slidably connected to the supporting assembly 10, that is, the sliding rod 11 may slide in the accommodating channel 101 along the length direction x of the sliding rod 11.

The second damping mechanism 17 is provided on a side of the supporting assembly 10. The first pressure spring 172 is located on an inner side of the pressure spring cover 173, that is, located on a side of the pressure spring cover 173 faces towards the sliding rod 11. The pressure spring cover 173 and the sliding rod 11 are configured to assist in limiting a stroke of the first pressure spring 172 to be along the length direction x of the sliding rod 11, and limit the first pressure spring 172 in the radial direction of the sliding rod 11. Meanwhile, the pressure spring cover 173 may be configured to protect the first pressure spring 172. In the length direction x of the sliding rod 11, the pressure spring cover 173 may cover the first pressure spring 172 and cover at least a portion of the first limit block 171. Meanwhile, in a radial direction of the sliding rod 11, the first limit block 171 is required to be located inside the pressure spring cover 173 to ensure that the sliding rod 11 may slide relative to the accommodating channel 101 in the length direction x of the sliding rod 11. The position of the pressure spring cover 173 is limited in the length direction x of the sliding rod 11 through the third clamping end 1731.

The first pressure spring 172 is located between the third clamping end 1731 of the pressure spring cover 173 and the first limit block 171, and the first pressure spring 172 is in a compressed state to maintain an elastic force on the first limit block 171, to make the first limit block 171 be in contact with a side wall of the third groove 111 away from the supporting assembly 10. When the sliding rod 11 receives hitting through the hitting cap 13, the sliding rod 11 moves relative to the supporting assembly 10 in the length direction x, and the sliding rod 11 drives the first limit block 171 to move towards a direction close to the supporting assembly 10, to make the sliding rod 11 compress the first pressure spring 172 through the first limit block 171, so that the impact axially received by the sliding rod 11 is at least partially absorbed by the first pressure spring 172, and thus a damping effect is achieved.

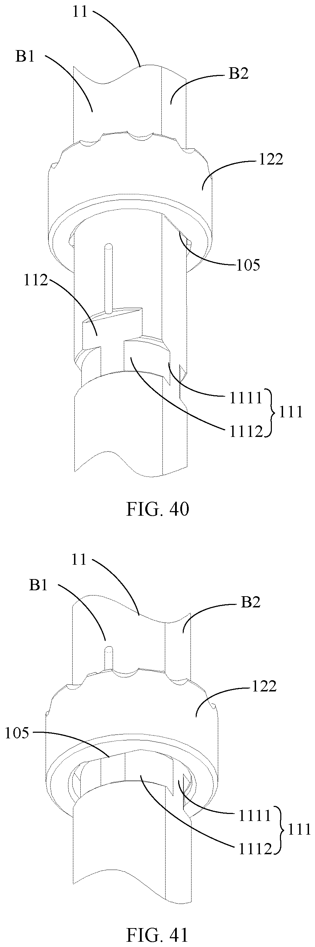

As shown in FIG. 40 and FIG. 41, in the above-mentioned embodiment, the sliding rod 11 may include a plane part B1 and an arc-surfaced part B2 connected to the plane part B1, the third groove 111 includes a first portion 1111 opposite to the plane part B1 and a second portion 1112 opposite to the arc-surfaced part B2, and the sliding rod 11 further includes a fourth groove 112 located on a side of the third groove 111 away from the supporting assembly 10. A size of the fourth groove 112 in the circumferential direction of the sliding rod 11 is smaller than a diameter of the sliding rod 11. In an assembling process, the sliding rod 11 is firstly slid into the pressure spring cover 173, then the first pressure spring 172 is slidably connected into the pressure spring cover 173, and the first clamping end 1721 is clamping-fitted with the third groove 111. Then, the first limit block 171 is slidably connected to the sliding rod 11, and during sliding, it is first ensured that the second clamping end 1711 is in contact with the plane part B1 and slides, when sliding to the third groove 111, because a diameter of the third groove 111 is relatively small, the first limit block 171 may be rotated in the circumferential direction of the sliding rod 11, and when rotating to a position corresponding to the fourth groove 112, the first limit block 171 slides into the fourth groove 112 under the elastic action of the first pressure spring 172, so that the second clamping end 1711 enters the fourth groove 112, and thus circumferential direction limit of the first limit block 171 is realized.

In a practicable embodiment, as shown in FIG. 37, the third damping mechanism 18 includes a second limit block 181, a limit pin 182 and a second pressure spring 183. The sliding rod 11 includes a first via hole 113, and the second limit block 181 includes second via holes 1811 that in one-to-one correspondence with two ends of the first via hole 113. The limiting pin 182 is insert and fitted with the first via hole 113 and the second via holes 1811, and the second pressure spring 183 is limited between the first damping mechanism 12 and the second limit block 181.

In the above-mentioned embodiment, the sleeve 121 may be formed with a fifth groove 114 for accommodating the second pressure spring 183, to limit the second pressure spring 183 in the circumferential direction of the sliding rod 11. In the above-mentioned embodiment, the limit pin 182 may be further welded and fixed to the second limit block 181, which is not particularly limited in the present application.

In the above-mentioned embodiment, the robotic arm may have a linear motion capability. In a linear motion mode, the robotic arm may also have the capability of moving along the length direction x while the sliding rod 11 moves in the length direction x. In this way, when the sliding rod 11 receives the hitting, the supporting assembly 10 will have a hysteresis movement state along with the sliding rod 11, that is, after the sliding rod 11 is hit and moves, the supporting assembly 10 moves linearly along the length direction x through the buffering of the second damping mechanism 17. During the linear movement of the supporting assembly 10, the supporting assembly 10 may compress the second pressure spring 183 to slide towards a direction close to the second limit block 181, so that movement of the robotic arm and force borne by the robotic arm are buffered.

The second pressure spring 183 in the third damping mechanism 18 also enables the first pressure spring 172 to remain in a compressed state without external force, ensuring that the sliding rod 11 has a predetermined positional relationship with the supporting assembly 10 when not being hit. In addition, no matter the sliding rod 11 moves relative to the supporting assembly 10 in a positive direction or a reverse direction of length direction x, the sliding rod 11 will not be in rigid contact with the supporting assembly 10, so that the impact of the sliding rod 11 is prevented from being directly transmitted to the supporting assembly 10.

In the above-mentioned embodiment, the second damping mechanism 17 may be provided at an end of the supporting assembly 10 close to the hitting cap 13, and the third damping mechanism 18 is provided at an end of the supporting assembly 10 close to the acetabular cup connecting mechanism 14. In the second damping mechanism 17, a size of the first pressure spring 172 in the length direction x of the sliding rod 11 is relatively long, so that the damping effect is better. In the third damping mechanism 18, a size of the second pressure spring 183 in the length direction x of the connecting section is relatively short, and a relative position of the second limit block 181 in the third damping mechanism 18 to the sliding rod 11 may be fixed, to reduce a sliding stroke of the supporting assembly 10 to the third damping mechanism 18 side, so that a space between the third damping mechanism 18 and the acetabular cup connecting mechanism 14 is increased, and thus facilitating a surgeon to hold the sliding rod 11 in this space.

In a practicable embodiment, the second damping mechanism 17 may be provided merely, and the second damping mechanism 17 may also be provided at a position of the third damping mechanism 18, which is not particularly limited in the present application.

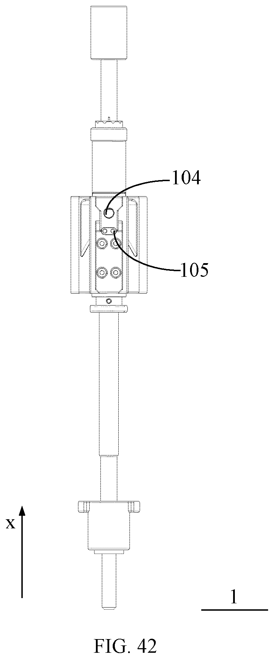

In a practicable embodiment, as shown in FIG. 36 and FIG. 42, the supporting assembly 10 further includes a locking pin spring 103, a locking pin 104, a contact 105, and a connecting block 106, and the supporting assembly 10 is configured to connect to the robotic arm. The locking pin spring 103, the locking pin 104 and the contact 105 all pass through via holes located in the connecting block 106, and are connected to a side wall of the accommodating channel 101.

In existing designs, the supporting assembly 10 and the robotic arm are rigidly connected together, such that the robotic arm is prone to be damaged or locking due to shock of the supporting assembly 10. The shock of the supporting assembly 10 mainly comes from the shock transmitted from the sliding rod 1 when it is hitting. In the actuator for installing the prosthesis 1 in the present disclosure, by providing the first damping mechanism 12, the second damping mechanism 17, and the third damping mechanism 18 between the supporting assembly 10 and the sliding rod 11, the shock transmitted to the supporting assembly 10 may be effectively reduced, so that effectively protecting the robotic arm, and thus reliability of the robotic arm is improved, ensuring that the surgery is performed smoothly, and thus loss in life and property is reduced.

FIG. 43 to FIG. 48 are structural schematic diagrams of an actuator for installing a prosthesis according to another embodiment. In this embodiment, the main difference between the actuator for installing the prosthesis and the actuator shown in FIG. 34 to FIG. 42 is the structure of the damping mechanism. The actuator for installing the prosthesis in this embodiment may be used in combination with the above-described prosthesis forming actuator.

Referring to FIG. 43 to FIG. 48 together, the actuator for installing the prosthesis includes a sliding rod 6, a supporting assembly 10, a damping apparatus, and a connecting mechanism 9. An end of the sliding rod 6 is configured to carry an acetabular prosthesis, and the other end is configured to receive impact to install an acetabular prosthesis to a target position.

An end of the connecting mechanism 9 is connected to the supporting assembly 10, and the other end of the connecting mechanism 9 is connected to an end of the robotic arm 1, so that the actuator for installing the prosthesis and the robot arm 1 are rigidly connected together. The sliding rod 6 is provided in an accommodating channel 101 of the supporting assembly 10 through the damping apparatus. The connecting mechanism 9 may be detachably connected to the robotic arm 1 through one or more manners, such as a bolt connection manner, a clamping manner, or the like.

In the present embodiment, an end of the sliding rod 6 is provided with a hitting cap 7, and the other end of the sliding rod 6 is provided with an acetabular cup connecting apparatus 8. The acetabular cup connecting apparatus is configured to connecting to an acetabular cup. The hitting cap 7 is a force-bearing position where the actuator for installing the prosthesis is hit by an external force during an acetabular cup implantation process in surgery, and also is the position where vibration is generated. The sliding rod 6 and the hitting cap 7 are rigidly connected together, and the vibration generated by the hitting is directly transferred to the sliding rod 6. The acetabular cup connecting apparatus 8 is mounted on the sliding rod 6, to rigidly connect the acetabular cup and the prosthesis installation actuator together.

The damping apparatus is configured to reduce the impact of the sliding rod 6 carrying the acetabular prosthesis on the supporting assembly 10. The damping apparatus includes a sleeve 5 and a buffering assembly 3. The sleeve 5 is configured to accommodate the sliding rod 6. The interior of the sleeve 5 is an accommodating channel 101. An inner diameter of the sleeve 5 is larger than a diameter of the sliding rod 6, and the two are coaxially arranged. A buffering assembly 3 includes a plurality of buffering elements, and the plurality of buffering elements are circumferentially provided in an accommodating space. An end of each of the buffering elements is connected to an outer circumference of the sleeve 5, and the other end of each of the buffering element is connected to an inner circumference of the accommodating channel 101.

When the sliding rod 6 is subjected to a force for installing the acetabular prosthesis, a portion of the impact borne by the sliding rod 6 is transmitted to the sleeve 5. The sleeve 5 impacts the buffering elements, and a buffering effect of the buffering elements may counteract a portion of the impact force, so that the impact on the supporting assembly and the robotic arm 1 is reduced.

The damping apparatus provided by the present invention transmits the impact received by the sliding rod 6 to the connecting mechanism, so that the vibration of the robotic arm is reduced, and thus the possibility of locking or failure of the robotic arm is reduced. This damping apparatus may effectively reduce the vibration transmitted to the robotic arm in any direction.

The buffering elements are constructed such that when the sliding rod 6 radially impacts the sleeve 5 and makes the sleeve 5 move radially, the buffering element is triggered and blocks a radial movement of the sleeve 5 w. A structure of the buffering elements is not unique, and the buffering element may be one or more of structures such as an elastic element, a sponge body or rubber. The buffering elements may block the radial movement of the sleeve 5 when the buffering elements are triggered, to play a certain buffering role.

In some optional embodiments, the buffering elements are the sponge bodies. Specifically, an end of the sponge body is connected to the sleeve 5, and the other end of the sponge body is connected to the supporting assembly. When the sliding rod 6 radially impacts the sleeve 5 and makes the sleeve 5 move radially, the sponge body may block the radial movement of the sleeve 5, and the sponge bodies with soft texture may play a certain buffering role on the impact.

In the present embodiment, the buffering elements are elastic members, the elastic members are circumferentially and evenly distributed in the accommodation space, and the elastic members may deform to absorb impact in an axial direction and/or a radial direction of the sleeve 5. The elastic members may be one or more of a spring, an elastic strips, an elastic pad, and an elastic airbag.

In some optional embodiments, the elastic members are springs disposed in a radial direction of the sleeve 5. An end of the springs is connected and fixed to the sleeve 5, and the other end are connected and fixed to the supporting assembly. The springs may generate elastic deformation and reset.

In the present embodiment, the number of the springs and the distribution of the springs may be adjusted according to requirements, and there may be four or more the springs evenly distributed in the circumferential direction. The elastic members may be cylindrical compression springs, and in order to connect stably without falling off, they may be tension springs, which are fixed between the supporting assembly and the sleeve 5 by a connecting structure such as a " hook ".

The springs may generate an overall deformation in the axial direction of the sliding rod 6. The springs may also extend and retract in the radial direction of the sliding rod 6.

In some optional embodiments, the elastic elements are elastic strips. The number of the elastic strips and the distribution of the elastic strips may be adjusted according to requirements. A plurality of the elastic strips may be evenly distributed along the axial direction of the sleeve 5.

In some optional embodiments, the elastic elements are elastic pads. The elastic pads may be rubber pads or plastic pads. A plurality of the elastic pads may be provided in the circumferential direction of the sleeve 5.

In some optional embodiments, the elastic elements are elastic airbags. There may be a plurality of the elastic airbags. An end of the elastic airbag is connected to the sleeve 5, and the other end is connected to the supporting assembly. When the sliding rod 6 radially impacts the sleeve 5 and make the sleeve 5 move radially, the elastic airbags may absorb the impact in the axial direction and the radial direction of the sleeve 5 at the same time.

As a specific implementation of the embodiment of the present invention, referring to FIG. 43 to FIG. 48 together, the damping apparatus further includes an axial buffering mechanism 2. The axial buffering mechanism 2 is axially provided between the sliding rod 6 and the sleeve 5. The sliding rod 6 is connected to the sleeve 5 through the axial buffering mechanism 2. The axial buffering mechanism 2 is configured to realize axial elastic positioning of the sliding rod 6 and the sleeve 5. When the sliding rod 6 is not subjected to an axial force, the axial buffering mechanism 2 enables the sliding rod 6 and the sleeve 5 to not move freely in the axial direction. When the sliding rod 6 is impacted by an external force, the sliding rod 6 and the sleeve 5 move axially relative to each other, and the axial buffering mechanism 2 is compressed. After the axial external force disappears, the axial buffering mechanism 2 drives the sliding rod 6 to reset.

In the present embodiment, the axial buffering mechanism 2 includes an axial elastic member, an end of the axial elastic member is connected to the sliding rod 6, the other end of the axial elastic member is connected to an end of the sleeve 5. The axial elastic member is configured to prevent the sliding rod 6 from being in rigid contact with the sleeve 5 and make the sliding rod 6 maintain or reset to a predetermined position relative to the sleeve 5.

In some optional embodiments, the axial elastic member may be an elastic bladder. The elastic bladder is in a ring structure. A through hole in the middle of the elastic bladder is used for the sliding rod 6 to pass through. The elastic bladder may be connected to the sliding rod 6 and the sleeve 5 through one or more of bonding, bolt connection and the like.

In the present embodiment, the elastic bladder is provided with two groups, which are respectively arranged at an upper end and a lower end of the sleeve 5, to improve reliability of the axial buffering mechanism 2.

In some optional embodiments, the axial buffering mechanism 2 includes a first buffering mechanism 21 and a second buffering mechanism 22. The first buffer mechanism 21 is provided on a side of the sleeve 5, and the first buffering mechanism 21 includes a first axial elastic member. An end of the first axial elastic member is connected to the sleeve 5, and the other end of the first axial elastic member is rigidly connected to the sliding rod 6. The second buffering mechanism 22 is provided on the other side of the sleeve 5, and the second buffering mechanism 22 includes a second axial elastic member. An end of the second axial elastic member is connected to the sleeve 5, and the other end of the second axial elastic member is rigidly connected to the sliding rod 6.

Specifically, both the first axial elastic member and the second axial elastic member are in a pre-compressed state.

The axial buffering mechanism 2 has a first use state and a second use state. When the sliding rod 6 receives an impact, the axial buffering mechanism 2 enters the first use state. The sliding rod 6 changes from a static state to motion in the axial direction, the axial buffering mechanism 2 is compressed or stretched, and a deformation resistance of the axial buffering mechanism 2 prevents the sliding rod from being in rigid contact with the sleeve, so that the impact of the sliding rod 6 is prevented from being completely transferred to the sleeve 5. When the impact on the sliding rod 6 is finished, the axial buffering mechanism 2 enters the second use state, and the axial buffering mechanism 2 drives the sliding rod 6 to reset, to make the sliding rod 6 be maintained at a predetermined position relative to the sleeve 5. That is, in the second use state, the sliding rod 6 is maintained in a relatively suspended middle position relative to the sleeve 5, without manually holding the sliding rod 6 or manually resetting the sliding rod 6.

In the present embodiment, the first buffering mechanism 21 includes a first limit block 211, a first elastic structure 212 and a cover 213. The first limit block 211 is provided on a side of the sleeve 5, and the first limit block 211 is rigidly connected to the sliding rod 6. An end of the first elastic structure 212 is connected to the cover 213, and the other end of the first elastic structure 212 is connected to the first limit block 211. An end of the cover 213 is movably connected to one end of the sleeve 5, and the other end is connected to the first elastic structure 212. The cover 213 is provided with a through hole adapted to the sliding rod 6. The cover 213 may move in the axial direction of the sliding rod 6. The cover 213 is provided with a connecting part. The connecting part is a pin body. The sleeve 5 is provided with a groove adapted to the pin body. A flat surface is provided on a portion of the structure of the sliding rod 6. A wall surface of the through hole of the cover 213 is composed of an arc-shaped surface and a flat surface. The flat surface of the through hole is adapted to the flat surface of the sliding rod 6.

In the present embodiment, the second buffering mechanism 22 includes a second limit block 221 and a second elastic structure 222, the second limit block 221 is provided on a side of the sleeve 5, and the second limit block 221 is rigidly connected to the sliding rod 6. An end of the second elastic structure 222 is connected to the sleeve 5, and the other end is connected to the second limit block 221.

As a specific implementation of the embodiment of the present invention, referring to FIG. 43 to FIG. 48, center lines of the sleeve 5, the buffering element, the first buffering mechanism 21 and the second buffering mechanism 22 coincides with an axis of the sliding rod 6.

The working principle of the acetabulum damping apparatus is as follow.

In a working state, the first limit block 211 and the second limit block 221 are rigidly connected to the sliding rod 6 without sliding and rotation. The first elastic structure 212 and the second elastic structure 222 are both in a compressed state. The sleeve 5 is in shaft-hole fit with a shaft hole of the sliding rod 6, and is pressed in the middle by the first elastic structure 212 and the second elastic structure 222. The sleeve 5 may slide up and down within a stroke range of the first elastic structure 212 and the second elastic structure 222. The cover 213 is combined with the sliding rod 6 by the arc-shaped surface, and is fit with the sliding rod 6 by the flat surface, so that the cover 213 may slide up and down and cannot rotate. The cover 213 is fit with the sleeve 5 through a pin-groove structure. In the working state, the pin groove is tightly pressed and cannot move out, so that the sleeve 5 cannot rotate in the circumferential direction.

When the hitting cap 7 is subjected to a force for installing the acetabular prosthesis, a portion of impact received by the sliding rod 6 causes the sliding rod 6 to move axially. The other portion of the impact received by the sliding rod 6 is transferred to the sleeve 5 through the axial buffering mechanism 2. Specifically, the sliding rod 6 axially moves to drive the axial buffering mechanism 2 to slide along the axial direction of the sliding rod 6 within a certain range of a stroke according to a designed stroke. More specifically, when the sliding rod 6 moves downwards, the first limit block 211 is driven to move downwards, and the first limit block 211 moves downwards to compress the first elastic structure 212, so that the first elastic structure 212 is subjected to a downward acting force. At the same time, the sliding rod 6 moves downward to drive the second limit block 221 to move downwards, the second limit block 221 moves downwards to enable the second elastic structure 222 to receive a downward acting force, and the second elastic structure 222 is stretched relative to an initial state. Through cooperation of the first buffering mechanism 21 and the second buffering mechanism 22, certain buffer may be generated in the axial direction. After the sleeve 5 is subjected to a force, the force is transmitted to the buffering assembly 3. When the buffering assembly 3 is a spring, a buffering effect may be generated in both the axial direction and the radial direction at the same time, that is, the buffer assembly 3 can effectively reduce axial and radial vibration transmitted by the sliding rod 6 to the connecting mechanism 9, and thereby vibration transmitted to the robotic arm 1 is weakened. Specifically, the number of the spring may be eight, and the eight springs are evenly mounted and fixed into a cavity between the sleeve 5 and the connecting mechanism 9. The eight buffering elements are of a same specification, and a size of the mounting structure is also consistent, so that the sleeve 5 and a housing portion of the connecting mechanism 9 are coaxially connected in theory. The hitting cap 7, the sliding rod 6 and the acetabular cup connecting apparatus 8 are stressed synchronously and move synchronously along the axial direction of the sliding rod 6.

Though the present disclosure has been described in detail as above with the general description and specific embodiments, it is possible to make some modification or improvement thereto based on the present disclosure, which will be obvious to a person skilled in the art. Therefore, such modification or improvement made based thereon, without departing from the spirit of the present disclosure, will fall within the protection scope of the present disclosure.

## Claims

1. An actuator for hip replacement, which is configured to prepare a space for installing a prosthesis on a bone and implant the prosthesis, comprising:
a first actuator, configured to connect a cutting tool to process an acetabulum and/or a medullary cavity, the first actuator having a first connector and a second connector; and
a second actuator, configured to connect to the second connector of the first actuator during an operation of implantation of the prosthesis is performed, and connect the prosthesis and receive impact produced during installing the prosthesis,
wherein the actuator for the hip replacement is configured to be installed to a robotic arm via the first connector.

2. The actuator for the hip replacement according to claim 1, wherein when the second actuator is connected to the first actuator, a structure to be connected to the prosthesis is parallel to a structure to be connected to the cutting tool.

3. The actuator for the hip replacement according to claim 1, wherein the first connector and the second connector are distributed on two ends of the first actuator.

4. The actuator for the hip replacement according to claim 1, wherein the first actuator is provided with a handle, and the handle is configured to be parallel to or coaxial with the cutting tool when the cutting tool is connected to the first actuator.

5. The actuator for the hip replacement according to claim 4, wherein when the cutting tool is connected to the first actuator, the handle and the cutting tool are distributed on two sides of the first actuator.

6. The actuator for the hip replacement according to any one of claims 1 to 4, wherein the first actuator comprises a power apparatus and a tool assembly which are detachably connected to each other.

7. The actuator for the hip replacement according to claim 5 or claim 6, wherein the first connector is provided on the power apparatus.

8. The actuator for the hip replacement according to claim 5 or claim 6, wherein the power apparatus comprises a built-in power assembly, the power assembly comprises a power source and an output shaft, and the output shaft is connected to the power source,
the tool assembly comprises a connecting part and a surgical tool, the surgical tool is rotatably provided on the connecting part, and the tool assembly is detachably provided on the power apparatus by the connecting part; and
when the tool assembly is connected to the power apparatus through the connecting part, the surgical tool forms a joint with the output shaft to receive rotation movement output by the output shaft.

9. The actuator for the hip replacement according to claim 8, wherein the joint is formed by an inserting action or a sleeving action of the surgical tool along an axial direction relative to the output shaft.

10. The actuator for the hip replacement according to claim 9, wherein the surgical tool and the output shaft are constructed to be in a spline connection.

11. The actuator for the hip replacement according to claim 8, wherein a radial positioning structure is further provided between the surgical tool and the power apparatus.

12. The actuator for the hip replacement according to claim 11, wherein the radial positioning structure is provided between the surgical tool and the output shaft.

13. The actuator for the hip replacement according to claim 11, wherein the radial positioning structure is a shaft-hole fit between the output shaft and the surgical tool.

14. The actuator for the hip replacement according to claim 8, wherein the connecting part is connected to the power apparatus through a rotating joint structure, to form an axial and circumferential direction limit to the connecting part.

15. The actuator for the hip replacement according to claim 14, wherein the rotating joint structure comprises a spiral groove provided on a circumferential face and a positioning pin, and the spiral groove is used for guiding the positioning pin and comprises a limiting part for limiting an axial direction and a circumferential direction of the positioning pin.

16. The actuator for the hip replacement according to claim 15, wherein the spiral groove is provided on the power apparatus, and the positioning pin is provided on the connecting part.

17. The actuator for the hip replacement according to claim 15, wherein the spiral groove comprises a rotating section and a positioning section, after the positioning pin enters the positioning section along the rotating section, the connecting part and the power apparatus have a circumferential positioning relationship and an axial positioning relationship.

18. The actuator for the hip replacement according to claim 17, wherein a positioning module is provided between the connecting part and the power apparatus, to form a predetermined acting force between the connecting part and the power apparatus.

19. The actuator for the hip replacement according to claim 18, wherein the positioning module comprises an elastic member, the elastic member is compressed by the power apparatus and the tool assembly to generate the predetermined acting force, and a direction of the predetermined acting force is an axial direction of the output shaft.

20. The actuator for the hip replacement according to claim 19, wherein the elastic member is provided between the surgical tool and the connecting part in the tool assembly, the elastic member compresses the surgical tool to make the surgical tool be axially compressed with the output shaft.

21. The actuator for the hip replacement according to claim 8, wherein the surgical tool is an acetabulum grinding file rod or a medullary cavity reamer.

22. The actuator for the hip replacement according to claim 6, further comprising a tracing assembly, and the tracing assembly being provided on a surface of the power apparatus.

23. The actuator for the hip replacement according to any one of claims 1 to 4, wherein when the first actuator is connected to an end section of the robotic arm, the first actuator forms an extension of the end section.

24. The actuator for the hip replacement according to any one of claims 1 to 4, wherein the second actuator is a prosthesis installing actuator, which comprises:
a sliding rod, a first end of the sliding rod is configured to connect the prosthesis, and a second end of the sliding rod is configured to receive the impact produced during installing the prosthesis;
a supporting assembly, comprising an accommodating channel, wherein the accommodating channel accommodates a portion of a rod section of the sliding rod, and the sliding rod is axially movable with respect to the supporting assembly; and the supporting assembly is used for connecting the prosthesis installing actuator to the second connector of the first actuator, and
a tracer, which is provided on the sliding rod to indicate an orientation of the sliding rod.

25. The actuator for the hip replacement according to claim 24, further comprising an axial buffering mechanism, and when the sliding rod is subjected to axial impact, the axial buffering mechanism forms axial buffer between the sliding rod and the supporting assembly.

26. The actuator for the hip replacement according to claim 25, wherein an axial limit structure is provided between the sliding rod and the supporting assembly, and the buffering mechanism is provided between the supporting assembly and the axial limit structure.

27. The actuator for the hip replacement according to claim 26, wherein the axial buffering mechanism is pre-compressed or pre-stretched.

28. The actuator for the hip replacement according to claim 26, wherein the accommodating channel is a channel penetrating through the supporting assembly, and the axial buffering mechanism comprises two buffering members which are provided on two ends of the channel, respectively.

29. The actuator for the hip replacement according to claim 28, wherein the two buffering members are both in a compressed state.

30. The actuator for the hip replacement according to claim 28, wherein the axial limit structure comprises a check ring provided on the sliding rod, and the buffering members are provided between the check ring and the supporting assembly.

31. The actuator for the hip replacement according to claim 30, wherein the axial limit structure further comprises an insulating member on a side of the supporting assembly, and the buffering members are provided between the insulating member and the check ring.

32. The actuator for the hip replacement according to claim 24, wherein the sliding rod further comprises a holding part for an operator to hold.

33. The actuator for the hip replacement according to claim 32, wherein the holding part is provided between the supporting assembly and the prosthesis.

34. The actuator for the hip replacement according to claim 32, wherein the holding part is rigidly connected to the sliding rod in an axial direction.

35. The actuator for the hip replacement according to claim 24, wherein a quick release mechanism is provided between the supporting assembly and the robotic arm, and the prosthesis installing actuator is connected to the robotic arm by the quick release mechanism.

36. The actuator for the hip replacement according to claim 35, wherein the quick release mechanism comprises a first limit mechanism and a second limit mechanism, and the second limit mechanism is a mechanism for manually removing a limit.

37. The actuator for the hip replacement according to claim 24, further comprising an adjusting assembly for adjusting a circumferential position of the prosthesis with respect to the sliding rod, and the adjusting assembly comprises:
an adapter shaft, an end of the adapter shaft being used for connecting with the prosthesis; and
an adjusting member, which is used for connecting the adapter shaft to the sliding rod, a circumferential position of the adjusting member is adjustable with respect to the sliding rod, and a circumferential position of the adjusting member with respect to the adapter shaft is fixed.

38. The actuator for the hip replacement according to claim 37, wherein the adjusting member is movable between a first position and a second position of the adapter shaft, a circumferential position of the adjusting member with respect to the sliding rod is fixed at the first position, and the circumferential position of the adjusting member with respect to the sliding rod is adjustable at the second position.

39. The actuator for the hip replacement according to claim 38, wherein a spline fit is formed between the adjusting member and the sliding rod at the first position, and/or the adjusting member and the adapter shaft are fitted with each other by a clamping block and a clamping groove, and the clamping groove extends along an axial direction of the adapter shaft.

40. The actuator for the hip replacement according to claim 37, further comprising a holding member, the holding member is constructed to keep the adjusting member in a first position when the adjusting member is not subjected to an external force.

41. The actuator for the hip replacement according to claim 24, further comprising a first damping mechanism; an inner diameter of the accommodating channel of the supporting assembly is larger than a diameter of the sliding rod, the first damping mechanism is provided on the supporting assembly and is connected to the sliding rod, the first damping mechanism is constructed to make the sliding rod be elastically held in the accommodating channel, and make a predetermined gap be formed between the sliding rod and the accommodating channel.

42. The actuator for the hip replacement according to claim 41, wherein the first damping mechanism has at least ability of radial elastic deformation, to allow the sliding rod to deflect or radially move in the accommodating channel.

43. The actuator for the hip replacement according to claim 41, wherein the first damping mechanism comprises a first elastic member having ability to deform along a radial direction and/or an axial direction of the accommodating channel.

44. The actuator for hip replacement according to claim 42 or claim 43, wherein the first damping mechanism further comprises a sleeve, an inner periphery of the sleeve is in shaft-hole fit with a shaft hole of the sliding rod, and an outer periphery of the sleeve is fitted with the first elastic member.

45. The actuator for the hip replacement according to claim 44, wherein the first elastic member is a damping pad, and the damping pad is in a ring shape and has a predetermined axial thickness and radial thickness.

46. The actuator for the hip replacement according to claim 45, wherein an outer circumference of the damping pad forms a shaft-hole fit with the accommodating channel, and an inner circumference of the damping pad forms a shaft-hole fit with the sleeve.

47. The actuator for the hip replacement according to claim 45, wherein a first circumferential direction limit structure is formed between the damping pad and the sleeve, and the first circumferential direction limit structure comprises a first groove and a first protrusion which are fit with each other; and/or,
a second circumferential direction limit structure is formed between the damping pad and the supporting assembly, and the second circumferential direction limit structure comprises a second groove and a second protrusion which are fit each other.

48. The actuator for the hip replacement according to claim 45, wherein the first damping mechanism further comprises an axial limit structure, and the axial limit structure is configured to prevent the first damping mechanism from axially moving out of the accommodating channel.

49. An actuator for installing a prosthesis, which is configured to perform a surgical plan under the hold of a robotic arm, comprising:
a sliding rod, a first end of the sliding rod is configured to connect the prosthesis, and a second end of the sliding rod is configured to receive impact produced during installing the prosthesis;
a supporting assembly, comprising an accommodating channel, wherein the accommodating channel accommodates a portion of a rod section of the sliding rod, and the sliding rod is axially movable with respect to the supporting assembly; and the supporting assembly is configured to connect the actuator for installing the prosthesis to an end of the robotic arm; and
a tracer, provided on the sliding rod to indicate an orientation of the sliding rod.

50. The actuator for installing the prosthesis according to claim 49, further comprising a first damping mechanism; an inner diameter of the accommodating channel is larger than a diameter of the sliding rod; the first damping mechanism is provided on the supporting assembly and is connected to the sliding rod, the first damping mechanism is constructed to make the sliding rod be elastically held in the accommodating channel, and make a predetermined gap be formed between the sliding rod and the accommodating channel.

51. The actuator for installing the prosthesis according to claim 50, wherein the first damping mechanism has at least ability of radial elastic deformation, to allow the sliding rod to deflect or radially move in the accommodating channel.

52. The actuator for installing the prosthesis according to claim 50 or claim 51, wherein the first damping mechanism comprises a first elastic member having ability to deform along a radial direction and/or an axial direction of the accommodating channel.

53. The actuator for installing the prosthesis according to claim 52, wherein the first damping mechanism further comprises a sleeve, an inner periphery of the sleeve is in shaft-hole fit with a shaft hole of the sliding rod, and an outer periphery of the sleeve is fitted with the first elastic member.

54. The actuator for installing the prosthesis according to claim 53, wherein the first elastic member is a damping pad, and the damping pad is in a ring shape and has a predetermined axial thickness and radial thickness.

55. The actuator for installing the prosthesis according to claim 54, wherein an outer circumference of the damping pad is in shaft-hole fit with the accommodating channel, and an inner circumference of the damping pad is in shaft-hole fit with the sleeve.

56. The actuator for installing the prosthesis according to claim 54, wherein a first circumferential direction limit structure is formed between the damping pad and the sleeve, and the first circumferential direction limit structure comprises a first groove and a first protrusion which are fit with each other; and/or,
a second circumferential direction limit structure is formed between the damping pad and the supporting assembly, and the second circumferential direction limit structure comprises a second groove and a second protrusion which are fit with each other.

57. The actuator for installing the prosthesis according to claim 54, wherein the first damping mechanism further an axial limit structure, and the axial limit structure is configured to prevent the first damping mechanism from axially moving out of the accommodating channel.

58. The actuator for installing the prosthesis according to claim 49, further comprising an axial buffering mechanism, and when the sliding rod is subjected to axial impact, the axial buffering mechanism forms axial buffer between the sliding rod and the supporting assembly.

59. The actuator for installing the prosthesis according to claim 58, wherein an axial limit structure is provided between the sliding rod and the supporting assembly, and the buffering mechanism is provided between the supporting assembly and the axial limit structure.

60. The actuator for installing the prosthesis according to claim 59, wherein the axial buffering mechanism is pre-compressed or pre-stretched.

61. The actuator for installing the prosthesis according to claim 59, wherein the accommodating channel is a channel penetrating through the supporting assembly, and the axial buffering mechanism comprises two buffering members which are provided on two ends of the channel, respectively.

62. The actuator for installing the prosthesis according to claim 61, wherein the two buffering members are both in a compressed state.

63. The actuator for installing the prosthesis according to claim 61, wherein the axial limit structure comprises a check ring provided on the sliding rod, and the buffering members are provided between the check ring and the supporting assembly.

64. The actuator for installing the prosthesis according to claim 63, wherein the axial limit structure further comprises an insulating member on a side of the supporting assembly, and the buffering members are provided between the insulating member and the check ring.

65. The actuator for installing the prosthesis according to claim 57, wherein the sliding rod further comprises a holding part for an operator to hold.

66. The actuator for installing the prosthesis according to claim 65, wherein the holding part is provided between the supporting assembly and the prosthesis.

67. The actuator for installing the prosthesis according to claim 65, wherein the holding part is rigidly connected to the sliding rod in an axial direction.

68. A joint forming actuator, comprising:
a power apparatus, comprising a robot connecting end and a built-in power assembly, the robot connecting end being configured to connect to an end of a robotic arm of a robot, the power assembly comprising a power source and an output shaft, and the output shaft being connected to the power source;
a tool assembly, comprising a connecting part and a surgical tool, the surgical tool being rotatably provided on the connecting part, and the tool assembly being detachably provided on the power apparatus through the connecting part; and
when the tool assembly is connected to the power apparatus through the connecting part, the surgical tool being joined to the output shaft to receive rotation movement output by the output shaft.

69. The joint forming actuator according to claim 68, wherein the joint is formed by an inserting action or a sleeving action of the surgical tool along an axial direction relative to the output shaft.

70. The joint forming actuator according to claim 69, wherein the surgical tool and the output shaft are constructed to be in a spline connection.

71. The joint forming actuator according to claim 68, wherein a radial positioning structure is further provided between the surgical tool and the power apparatus.

72. The joint forming actuator according to claim 71, wherein the radial positioning structure is provided between the surgical tool and the output shaft.

73. The joint forming actuator according to claim 71, wherein the radial positioning structure is a shaft-hole fit between the output shaft and the surgical tool.

74. The joint forming actuator according to claim 68, wherein the connecting part is connected to the power apparatus through a rotating joint structure, to form an axial and circumferential direction limit to the connecting part.

75. The joint forming actuator according to claim 74, wherein the rotating joint structure comprises a spiral groove provided on a circumferential face and a positioning pin, and the spiral groove is used for guiding the positioning pin and comprises a limiting part for limiting an axial direction and a circumferential direction of the positioning pin.

76. The joint forming actuator according to claim 75, wherein the spiral groove is provided on the power apparatus, and the positioning pin is provided on the connecting part.

77. The joint forming actuator according to claim 75, wherein the spiral groove comprises a rotating section and a positioning section, after the positioning pin enters the positioning section along the rotating section, the connecting part and the power apparatus have a circumferential positioning relationship and an axial positioning relationship.

78. The joint forming actuator according to claim 77, wherein a positioning module is provided between the connecting part and the power apparatus, to form a predetermined acting force between the connecting part and the power apparatus.

79. The joint forming actuator according to claim 78, wherein the positioning module comprises an elastic member, the elastic member is compressed by the power apparatus and the tool assembly to generate the predetermined acting force, and a direction of the predetermined acting force is an axial direction of the output shaft.

80. The joint forming actuator according to claim 79, wherein the elastic member is provided between the surgical tool and the connecting part in the tool assembly, the elastic member compresses the surgical tool to make the surgical tool be axially compressed with the output shaft.

81. The joint forming actuator according to claim 68, wherein the surgical tool is an acetabulum grinding file rod or a medullary cavity reamer.

82. The joint forming actuator according to claim 68, further comprising a tracing assembly, and the tracing assembly being provided on a surface of the power apparatus.

83. The joint forming actuator according to claim 68, wherein when the power apparatus is configured to form an extension of an end section of the robotic arm when connected to the end section, and the output shaft is transverse to the end section.

84. The joint forming actuator according to claim 68, wherein the power apparatus further comprises a connector for an actuator for installing a prosthesis.

85. The joint forming actuator according to claim 84, wherein the robot connecting end and the actuator for installing the prosthesis are distributed on two ends of the power apparatus.

86. The joint forming actuator according to claim 68, wherein the power apparatus further comprises a handle configured to be substantially parallel to a rod connecting the surgical tool.

87. The joint forming actuator according to claim 86, wherein the handle and the surgical tool are distributed on two sides of the power apparatus.

88. A tool assembly, which is configured to be connected and driven by a power apparatus to machine a joint, comprising a connecting part and a surgical tool, the surgical tool being rotatably provided on the connecting part, the connecting part being configured to detachably connect the tool assembly to the power apparatus, and
when the tool assembly is connected to the power apparatus through the connecting part, the surgical tool forming a joint with an output shaft of the power apparatus to receive rotation movement output by the output shaft.

89. The tool assembly according to claim 88, wherein the joint is formed by an inserting action or a sleeving action of the surgical tool along an axial direction of relative to the output shaft.

90. The tool assembly according to claim 89, wherein the surgical tool has a spline structure to form a spline connection with the output shaft.

91. The tool assembly according to claim 88, wherein the surgical tool is provided with a radial positioning part to form a radial positioning structure with the power apparatus.

92. The tool assembly according to claim 91, wherein the radial positioning part is a positioning hole.

93. The tool assembly according to claim 88, wherein the connecting part is provided with a rotating joint part to form a rotating joint structure with the power apparatus, and the rotating joint structure is configured to form an axial and circumferential direction limit to the connecting part.

94. The tool assembly according to claim 93, wherein the rotating joint part is a spiral groove provided on a circumferential face or a positioning pin.

95. The tool assembly according to claim 88, wherein the connecting part is provided with a positioning module, and the positioning module make a predetermined acting force be formed between the connecting part and the power apparatus.

96. The tool assembly according to claim 95, wherein the positioning module comprises an elastic member, the elastic member is compressed by the power apparatus and the tool assembly to generate the predetermined acting force, and a direction of the predetermined acting force is an axial direction of the output shaft.

97. The tool assembly according to claim 96, wherein the elastic member compresses the surgical tool to make the surgical tool be axially compressed with the output shaft.

98. The tool assembly according to claim 88, wherein the surgical tool is an acetabulum grinding file rod or a medullary cavity reamer.

99. The tool assembly according to claim 88, wherein the connecting part is further configured to position the tool assembly to the power apparatus.

100. A surgical system, comprising:
an actuator which is the actuator for the hip replacement according to any one of claims 1 to 48;
a robotic arm, connected to the first connector of the actuator,
a navigation system, configured to measure a position of the actuator, and
a controlling system, configured to drive the robotic arm to move the actuator to a target position according to a surgical plan.
